# EUROPEAN PATENT APPLICATION

(11) **EP 1 938 803 A2**
(43) Date of publication of application: **02.07.2008**
(21) Application number: 08006465.2
(22) Date of filing: 27.02.2004
(51) Int. Cl.: A61K 9/14, A61K 31/5415

(54) **Formulations comprising nanoparticulate meloxican**

(30) Priority: 03.03.2003 US 450705 P
(62) Divisional of application: 04785761.0
(71) Applicant: Elan Pharma International Limited, Dublin 2 (IE)
(72) Inventor: Cooper, Eugene, R., Berwyn PA 19312 (US); Ryde, Tuula, Malvern PA 19355 (US); Pruitt, John, D., Suwanee GA 30024 (US); Kline, Laura, Harleysville PA 19438 (US)
(74) Representative: Wichmann, Hendrik

(57) **Abstract**

The present invention is directed to compositions comprising meloxicam. The stable meloxicam compositions comprise (a) nanoparticulate particles of meloxicam having an effective average particle size of less than about 2000 nm, (b) particles of meloxicam having an effective average particle size which is less or greater than about 2 microns; wherein said effective average particle size is different than the particle size of the nanoparticulate particles of meloxicam (a); and (c) at least one surface stabilizer. The invention relates also to uses of the composition.

## Description

### FIELD OF THE INVENTION

The present invention relates to nanoparticulate compositions comprising meloxicam and at least one surface stabilizer adsorbed to or associated with the surface of the drug. The nanoparticulate meloxicam particles have an effective average particle size of less than about 2000 nm.

### BACKGROUND OF THE INVENTION

### A. Background Regarding Nanoparticulate Active Agent Compositions

Nanoparticulate active agent compositions, first described in U.S. Patent No. 5,145,684 ("the '684 patent"), are particles consisting of a poorly soluble therapeutic or diagnostic agent having adsorbed onto the surface thereof a non-crosslinked surface stabilizer. The '684 patent does not describe nanoparticulate compositions of meloxicam.

Methods of making nanoparticulate active agent compositions are described, for example, in U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate active agent compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast. Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate," 6,428,814 for "Bioadhesive Nanoparticulate Compositions Having Cationic Surface Stabilizers;" 6,431,478 for "Small Scale Mill;" 6,432,381 for "Methods for Targeting Drug Delivery to the Upper and/or Lower Gastrointestinal Tract," 6,582,285 for "Apparatus for Sanitary Wet Milling," 6,592,903 for "Nanoparticulate Dispersions Comprising a Synergistic Combination of a Polymeric Surface Stabilizer and Dioctyl Sodium Sulfosuccinate," and 6,656,504 for "Nanoparticulate Compositions Comprising Amorphous Cyclosporine and Methods of Making and Using Such Compositions," all of which are specifically incorporated by reference.

In addition, U.S. Patent Application No. 20020012675 A1, published on January 31, 2002, for "Controlled Release Nanoparticulate Compositions," and WO 02/098565 for "System and Method for Milling Materials," describe nanoparticulate active agent compositions, and are specifically incorporated by reference. None of these references describe nanoparticulate compositions of meloxicam.

U.S. Patent Application No. 20020028238, published on March 7, 2002, for "Use of a Celecoxib Composition for Fast Pain Relief;" U.S. Patent Application No. 20020006951, published on January 17, 2002, for "Solid State Form of Celecoxib Having Enhanced Bioavailability;" and U.S. Patent Application No. 20020019431, published on February 14, 2002, for "Porous Celecoxib Matrices and Methods of Manufacture Thereof," describe nanoparticulate celecoxib compositions.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter."

### B. Background Regarding Meloxicam

Meloxicam, also known as 4-hydroxy-2-methyl-N-(5-methyl-2-thiazolyl)-2-H-1,2-benzothiazine-3-carboxamide 1,1-dioxide, is a member of the enolic acid group of nonsteroidal anti-inflammatory drugs (NSAIDs). Meloxicam is an oxicam derivative with the following chemical structure: 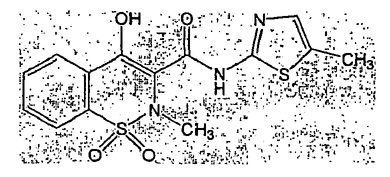 Meloxicam has an empirical formula of C₁₄H₁₃N₃O₄S₂ and a molecular weight of 351.41. *See* The Physicians' Desk Reference, 56th Ed., pp. 1054 (2002); and The Merck Index, 13th Ed., pp. 1040-1041 (Merck & Co. 2001). Meloxicam is practically insoluble in water with higher solubility observed in strong acids and bases. It is very slightly soluble in methanol. The Physicians' Desk Reference, 56th Ed., pp. 1054.

4-hydroxy-2H-1,2-benzothiazine-3-carboxamide-1,1-dioxides and salts thereof, as well as methods of preparing these compounds, pharmaceutical compositions containing them as active ingredients, and methods of using them as antiphlogistics, are discussed in U.S. Patent No. 4,233,299. *See also* German Patent No. 2,756,113. The pharmacology of meloxicam in horses is discussed in Lees et al., Brit. Vet. J., 147: 97 (1991); veterinary trials in dogs are discussed in Henderson et al., Prakt. Tierarzt., 75:179 (1994); the physiochemical properties of meloxicam are discussed in Tsai et al., Helv. Chim. Acta, 76:842 (1993); the pharmacology, mechanism of action, and clinical efficacy are discussed in Brit. J. Rheumatol., 35(Suppl. 1):1-77 (1996); and clinical trials of gastrointestinal tolerability in arthritis is discussed in Hawkey et al., Brit. J. Rheumatol., 37:937 (1998), and Dequeker et al., Brit. J. Rheumatol., 37:946 (1998).

Meloxicam exhibits anti-inflammatory, analgesic, and antifebrile activities. Like other NSAIDs, the primary mechanism of action of meloxicam is via inhibition of the cyclooxygenase (COX) enzyme system resulting in decreased prostaglandin synthesis. *See* The Physicians' Desk Reference, 56th Ed., pp. 1054 (2002). The COX enzyme system is comprised of at least two isoforms of COX. COX-1 is constituatively expressed in the gastrointestinal tract and the kidneys and is involved in the production of prostaglandins required for gastric mucosal production and proper renal blood flow. *See* Vane et al., Proc. Natl. Acad. Sci. USA, 91:2046-2050 (1994); Oulette et al., Proc. Natl. Acad. Sci., 98:14583-14588 (2001); and Seibert et al., Proc. Natl. Acad. Sci., 91:12013-12017 (1994). In contrast, COX-2 is not present in healthy tissue and its expression is induced in certain inflammatory states. *Id.*

The pathological production of prostaglandins by COX-2 is implicated in a number of human disease states, including rheumatoid arthritis, osteoarthritis, pyrexia, asthma, bone resorption, cardiovascular diseases, nephrotoxicity, atherosclerosis, and hypotension. *Id.* Elevated levels of prostaglandins enhance or prolong pro-inflammatory signals which cause the pain, stiffness, and inflammation associated with these conditions. *See* Smith et al., Proc. Natl. Acad. Sci., 95:13313-13318 (1998).

Meloxicam is superior to traditional non-selective NSAIDs because it selectively inhibits COX-2, thus causing fewer gastrointestinal problems such as bleeding, heartburn, reflux, diarrhea, nausea, and abdominal pain. Meloxicam preferentially inhibits COX-2 with a COX-2/COX-1 inhibition ratio of 0.09. It is desirable to selectively inhibit COX-2 and the pathological production of prostaglandins for which that enzyme is responsible because the therapeutic analgesic/anti-inflammatory properties of NSAIDs occur by inhibition of inducible COX-2 at the site of inflammation. Conversely, the majority of adverse drug reactions to NSAIDs, including gastrointestinal ulcers and renal failure, result from inhibition of the consitutative COX-1 enzymes. This is because as a result of such COX-1 inhibition, prostaglandins necessary for gastric mucosal production and renal blood circulation are not produced. *See* Vane et al., Proc. Natl. Acad. Sci. USA, 91:2046 (1994); Oulette et al., Proc. Natl. Acad. Sci., 98:14583 (2001); and Seibert et al., Proc. Natl. Acad. Sci., 91:12013 (1994). Compounds that selectively inhibit the biosynthesis of prostaglandins by inhibiting the activity of the inducible enzyme, COX-2, exert anti-inflammatory effects without the adverse side effects associated with COX-1 inhibition.

Some of the trade names under which meloxicam has or is marketed include MOBIC®, MOBEC®, MOBICOX®, MOVALIS®, and MOVATEC®. Meloxicam has been shown to be useful in the symptomatic treatment of painful osteoarthritis (arthrosis, degenerative joint disease), symptomatic treatment of rheumatoid arthritis, symptomatic treatment of ankylosing spondylitis, and symptomatic treatment of the signs and symptoms of osteoarthritis, including pain, stiffness, and inflammation.

The form of meloxicam currently marketed in the United States is MOBIC® (Boehringer Ingelheim Pharmaceuticals, Inc., Ridgefield, CT), provided in 7.5 and 15 mg tablets. The bioavailability of a single 30 mg oral dose is 89% as compared to a 30 mg intravenous bolus injection. The pharmacokinetics of a single intravenous dose of meloxicam is dose-proportional in the range of 5 to 60 mg. *See* The Physicians' Desk Reference, 56th Ed., pp. 1054 (2002). After administration of multiple oral doses of meloxicam, the pharmacokinetics is dose-proportional in the range of 7.5 to 15 mg. The rate or extent of absorption is not affected by multiple dose administration. Under fasted steady state conditions, the mean Cₘₐₓ is achieved within four to five hours, with a second meloxicam concentration peak occurring at approximately twelve to fourteen hours postdose, which suggests gastrointestinal recirculation. Under steady state fed conditions in healthy adult males, the 7.5 mg tablets have a mean Cₘₐₓ of 1.05 µg/mL, a Tₘₐₓ of 4.9 hrs, and a t_{1/2} of 20.1 hours. Under steady state fed conditions in elderly males and females, the 15 mg tablets have a Cₘₐₓ of 2.3 and 3.2 µg/mL, respectively, a Tₘₐₓ of 5 and 6 hrs, respectively, and a t_{1/2} of 21 and 24 hrs, respectively. *See* The Physicians' Desk Reference, 56th Ed., pp. 1054 (2002).

Although meloxicam has been tested and approved by the FDA only for relief of the signs and symptoms of osteoarthritis, it may be useful in relieving the signs and symptoms of rheumatoid arthritis, lower back pain, and acute pain, e.g. treatment of post surgical pain, treatment of pain resulting from battle field wounds, and migraine headaches. Meloxicam may be especially effective for treatment of all types of pain associated with inflammation.

NSAIDs, like meloxicam, are useful in pain management because NSAIDs provide an analgesic effect without the sedation and addictive properties of narcotic analgesics. Furthermore, the long t_{1/2} of meloxicam makes it useful for long-lasting relief which is not provided by narcotic analgesics. However, due to their typically long onset of action, conventional NSAIDs, including conventional meloxicam, are frequently inappropriate for management of acute pain.

Because meloxicam is practically insoluble in water, attaining sufficient bioavailability of this drug is problematic. Prior art methods of increasing the bioavailability of meloxicam include increasing its solubility by forming a cyclodextrin complex of the drug (*see* U.S. Pat. No. 6.284,269) or by forming a salt of meloxicam with an inorganic or organic base (U.S. Pat. Appln. Pub. No. US 2002/0035107 A1).

Published U.S. Patent Application No. 20020035264, for "Ophthalmic Formulation of a Selective Cyclooxygenase-2 Inhibitory Drug," describes pharmaceutical compositions suitable for topical administration to an eye which contain a selective COX-2 inhibitory drug, or nanoparticles of a drug of low water solubility, in a concentration effective for treatment and/or prophylaxis of a disorder in the eye, and one or more ophthalmically acceptable excipients that reduce rate of removal from the eye such that the composition has an effective residence time of about 2 to about 24 hours. Examples of such ophthalmically acceptable excipients given in the published application include cross-linked carboxyl-containing polymers which form in situ gellable aqueous solution, suspension or solution/suspension. Such excipients, which are described in U.S. Pat. No. 5,192,535, can be undesirable. Moreover, this disclosure, whis is limited to ocular formulations, does not address a need for oral fast onset meloxicam formulations for treating migraine.

Published U.S. Patent Application No. 20020077328, for "Selective Cyclooxygenase-2 Inhibitors and Vasomodulator Compounds for Generalized Pain and Headache Pain," refers to a therapeutic combination useful in the treatment, amelioration, prevention, or delay of pain comprising a high energy form of a selective cyclooxygenase-2 inhibitor, a vasomodulator, and a pharmaceutically acceptable excipient, carrier, or diluent. The cyclooxygenase-2 inhibitor and vasomodulator are each being present in an amount effective to contribute to the treatment, prevention, ameloriation or delay of pain. Disclosed vasomodulators include vasoconstrictors, vasodilators, bronchodilation agents, and bronchoconstriction agents, such as rennin-angiotensin system antagonists, nitrovasodilators, direct vasodilators, calcium channel blocking drugs, phosphodiesterase inhibitors, sympathomimetics, sympatholytics, and nitric oxide synthase inhibitors. Such additional pharmaceutical agents can be undesirable, as they can cause unwanted side-effects.

There is a need in the art to provide a readily bioavailable form of meloxicam while avoiding the prior art methods of using solubilizing agents, as well as avoiding the prior art COX-2 inhibitor formulations having undesirable qualities. The present invention satisfies this need.

There is a need in the art for nanoparticulate meloxicam formulations which overcome these and other problems associated with prior conventional meloxicam formulations. The present invention satisfies these needs.

### SUMMARY OF THE INVENTION

The present invention relates to compositions comprising nanoparticulate meloxicam. The compositions comprise nanoparticulate meloxicam and at least one surface stabilizer adsorbed on or associated with the surface of the meloxicam particles. The nanoparticulate meloxicam particles, which have an effective average particle size of less than about 2000 nm, surprisingly exhibit superior Tₘₐₓ profiles as compared to conventional prior meloxicam formulations.

The present invention also relates to compositions of nanoparticulate meloxicam in combination with one or more other conventional or nanoparticulate active agents. If such other active agents are in a nanoparticulate form, then such other active agents will have one or more surface stabilizers adsorbed on or associated with to the surface of the active agent. The phrase "conventional active agents or drugs," as used in this application, refers to non-nanoparticulate active agents or drugs. Non-nanoparticulate active agents have an effective average particle size of greater than about 2 microns.

Another aspect of the invention is directed to pharmaceutical compositions comprising a nanoparticulate meloxicam composition of the invention. The pharmaceutical compositions preferably comprise nanoparticulate meloxicam, at least one surface stabilizer, and a pharmaceutically acceptable carrier, as well as any desired excipients. Alternatively, such pharmaceutical compositions can additionally comprise one or more non-meloxicam conventional or nanoparticulate active agents.

The nanoparticulate meloxicam formulations of the invention may require smaller doses as compared to prior conventional meloxicam formulations. In addition, the nanoparticulate meloxicam formulations of the invention may exhibit increased bioavailability, and superior Cₘₐₓ profiles as compared to conventional prior meloxicam formulations.

This invention further discloses a method of making a nanoparticulate meloxicam composition according to the invention. Such a method comprises contacting meloxicam and at least one surface stabilizer for a time and under conditions sufficient to provide a nanoparticulate meloxicam composition. The one or more surface stabilizers can be contacted with meloxicam either before, during, or after size reduction of the meloxicam. Alternatively, one or more non-meloxicam active agents can be reduced in size at the same time as meloxicam, to produce a nanoparticulate meloxican and nanoparticulate non-meloxicam active agent composition. A non-meloxicam active agent, which is either conventional or nanoparticulate sized, can also be added to the nanoparticulate meloxicam composition after size reduction.

The present invention is directed to methods of using the compositions of the invention in the treatment of, for example, pain, such as post-surgical pain, pain associated with battlefield wounds, and migraine headaches. The compositions of the invention can also be used in methods of treating the signs and symptoms of, for example, arthritis, pyrexia, asthma, bone resorption, cardiovascular diseases, nephrotoxicity, atherosclerosis, hypotension, arthrosis, ankylosing spondylitis, joint stiffness, lower back pain, pain associated with inflammation, inflammatory-related disorders, cancer, kidney disease, Alzheimer's disease, familial adenomatous polyposis, fever, acute mastitis, diarrhea, colonic adenomas, and tumors.

The compositions of the invention are useful in treating indications where anti-inflammatory agents, anti-angiogenesis agents, antitumorigenic agents, immunosuppressive agents, NSAIDs, COX-2 inhibitors, analgesic agents, anti-thrombotic agents, narcotics, or antifebrile agents are typically used.

Such methods comprise administering to a subject a therapeutically effective amount of a nanoparticulate meloxicam composition according to the invention. Alternatively, such methods comprise administering to a subject a therapeutically effective amount of a nanoparticulate meloxicam composition in combination with one or more non-meloxicam active agents. Such non-meloxicam active agents can be either conventional or nanoparticulate.

Both the foregoing general description and the following detailed description are exemplary and explanatory and are intended to provide further explanation of the invention as claimed. Other objects, advantages, and novel features will be readily apparent to those skilled in the art from the following detailed description of the invention.

### BRIEF DESCRIPTION OF THE FIGURE

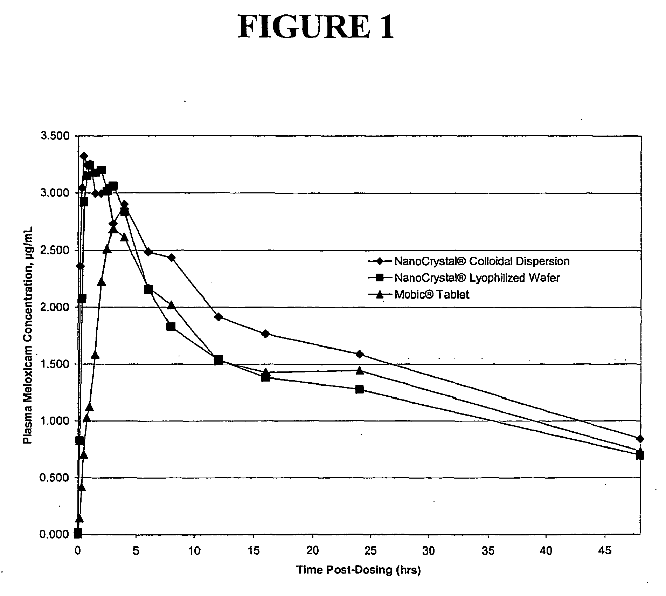

- Figure 1:: Shows the plasma concentration over time of meloxicam following administration of three different meloxicam formulations: a liquid dispersion of nanoparticulate meloxicam, a lyophilized wafer of nanoparticulate meloxicam, and a tablet of conventional non-nanoparticulate meloxicam, MOBIC® (Boehringer Ingelheim Pharmaceuticals, Inc.).

### DETAILED DESCRIPTION OF THE INVENTION

The present invention is directed to compositions comprising nanoparticulate meloxicam. The compositions comprise nanoparticulate meloxicam and at least one surface stabilizer adsorbed on or associated with the surface of the drug. The nanoparticulate meloxicam particles, which have an effective average particle size of less than about 2000 nm, surprisingly exhibit superior Tₘₐₓ profiles as compared to conventional prior meloxicam formulations.

As taught in the '684 patent, not every combination of surface stabilizer and active agent will result in a stable nanoparticulate composition. It was surprisingly discovered that stable nanoparticulate meloxicam formulations can be made. As described in more detail below, preferred surface stabilizers include polyvinylpyrrolidone (e.g., Kollidon® 12 PF, Kollidon^{®} 17 PF), docusate sodium , block polymers of polyethylene glycol and polypropylene glycol, poloxamers (*e.g.*, Pluronics F68^{®} and F108^{®}, which are block copolymers of ethylene oxide and propylene oxide), polyethylene sorbitan monooleate (Polysorbate 80), sodium Deoxycholate, lecithin, lysozyme, and mixtures thereof. Docusate sodium is particularly useful as a surface stabilizer in combination with one or more other surface stabilizers.

As described in Example 3 below, many of these surface stabilizers are particularly suited for injectable nanoparticulate meloxicam formulations. This is significant, and surprising, as for injectable formulations it is critical that very small meloxicam particles be obtained. Moreover, the composition must be stable, with very little or no particle size growth observed, as injectable formulations having large particles can cause embolism.

Nanoparticulate meloxicam formulations suitable parenteral injection (e.g., intravenous, intramuscular, or subcutaneous) for the treatment of acute pain are highly superior to conventional meloxicam formulations because they have much faster onset of action due to the nanoparticulate size of the active agent.

In addition to exhibiting dramatically superior Tₘₐₓ profiles, the nanoparticulate meloxicam formulations preferably also exhibit improved pharmacokinetic profiles as compared to conventional meloxicam formulations, resulting in faster onset of action and smaller effective doses as compared to prior conventional meloxicam formulations.

Conventional formulations of meloxicam are inappropriate for management of acute pain due to delayed onset of action, as such meloxicam formulations have a Tₘₐₓ of 4-6 hours, which is more than five times as long as most narcotic analgesic drugs. *See* The Physician's Desk Reference, 56th Ed., pp. 446 and 1054. Unlike conventional meloxicam formulations, nanoparticulate meloxicam formulations, which exhibit faster onset of action, are useful in treating acute pain where fast pain relief is required.

Additionally, any drug, including meloxicam, can have adverse side effects. Thus, lower doses of meloxicam which can achieve the same or better therapeutic effects as those observed with larger doses of conventional meloxicam are desired.

Nanoparticulate formulations of meloxicam also provide a longer duration of pain relief as compared to traditional narcotic analgesic drugs. While traditional narcotics provide fast onset of action, the duration of pain relief is short. Nanoparticulate meloxicam formulations combine the fast onset of traditional narcotics with the duration of pain relief of conventional NSAIDs. The long half-life of meloxicam, approximately 20 hours as compared to 2-3 hours for most narcotics, confers a long duration of action and thus requires less frequent dosing.

Additionally, nanoparticulate meloxicam formulations do not possess the sedative and addictive properties of narcotic analgesics. Meloxicam does not cause drowsiness and is not addictive, making it a preferred analgesic when ambulation is important or when treatment is protracted and chemical dependency could result from continued use of narcotic analgesics.

Nanoparticulate formulations can be prepared for oral administration for treatment of, for example, migraine headaches. The use of oral nanoparticulate formulations also provide much faster onset of action as compared to conventional orally dosed meloxicam formulations.

In addition, the invention encompasses compositions comprising nanoparticulate meloxicam, one or more surface stabilizers, and one or more non-meloxicam active agents, either conventional or nanoparticulate. Methods of using such combination compositions are also encompassed by the invention. For example, additional analgesic drugs can be used in combination with nanoparticulate meloxicam, such as one or more COX-2 inhibitors, NSAIDs, or narcotics. Other exemplary types of active agents which can be used in combination with nanoparticulate meloxicam are described below. If the non-meloxicam active agent is in nanoparticulate form, then such a non-meloxicam active agent also has one or more surface stabilizers adsorbed on or associated with the surface of the active agent. The surface stabilizer(s) adsorbed on or associated with the surface of the non-meloxicam active agent can be the same as or different from the surface stabilizer(s) adsorbed on or associated with the surface of the nanoparticulate meloxicam.

In general, such non-meloxicam active agents do not include vasomodulators, such as those described in U.S. Published Patent Application No. 20020077328.

In yet another embodiment of the invention, a first meloxicam formulation providing the pharmacokinetic profile required herein is co-administered with at least one other meloxicam formulation that generates a different pharmacokinetic profile, specifically one exhibiting slower absorption into the bloodstream and therefore a longer Tₘₐₓ, and typically a lower Cₘₐₓ. For example, the second meloxicam formulation can have a conventional particle size, which produces a longer Tₘₐₓ, and typically a lower Cₘₐₓ. Alternatively, a second, third, or fourth meloxicam formulation can differ from the first, and from each other, in the effective average particle sizes of each composition. The different particle sizes produce different Tₘₐₓₛ. The combination of fast pain relief provided by the first formulation and longer-lasting pain relief provided by the second (or third, fourth, etc.) formulation can reduce the dose frequency required.

Preferably where co-administration of a "fast-acting" formulation and a "longer-lasting" formulation is desired, the two formulations are combined within a single composition, for example a dual-release composition.

### A. Compositions

The invention provides compositions comprising nanoparticulate meloxicam particles and at least one surface stabilizer. The surface stabilizers are adsorbed on or associated with the surface of the meloxicam particles. Surface stabilizers useful herein physically adhere on the surface of the nanoparticulate meloxicam but do not chemically react with the meloxicam particles or itself. Individual molecules of the surface stabilizer are essentially free of intermolecular cross-linkages.

The invention also provides compositions of nanoparticulate meloxicam in combination with one or more conventional or nanoparticulate non-meloxicam drugs.

The present invention includes nanoparticulate meloxicam compositions together with one or more non-toxic physiologically acceptable carriers, adjuvants, or vehicles, collectively referred to as carriers. The compositions can be formulated for parenteral injection (e.g., intravenous, intramuscular, or subcutaneous), oral (in solid, liquid, or aerosol form), vaginal, nasal, rectal, ocular, local (powders, ointments or drops), buccal, intracisternal, intraperitoneal, or topical administration, and the like. Such compositions can also comprise one or more conventional or nanoparticulate non-meloxicam drugs.

The present invention provides compositions of meloxicam with a desirable pharmacokinetic profile when administered to mammalian subjects. Preferably, the Tₘₐₓ of a 7.5 mg orally administered dose of nanoparticulate meloxicam, when assayed in the plasma of a mammalian subject following administration of an initial dose, is less than about 5 hours, less than about 4 hours, less than about 3 hours, less than about 2 hours, less than about 1 hour, less than less than about 50 minutes, less than about 45 minutes, less than about 40 minutes, less than about 35 minutes, less than about 30 minutes, less than about 20 minutes, less than about 15 minutes, less than about 10 minutes, or less than about 5 minutes.

In addition, preferably the Cₘₐₓ of a 7.5 mg orally administered dose of nanoparticulate meloxicam, when assayed in the plasma of a mammalian subject following administration of an initial dose, is greater than about 1 µg/mL, greater than about 3 µg/mL, greater than about 5 µg/mL, greater than about 10 µg/mL, or greater than about 15 µg/mL.

The desirable pharmacokinetic profile, as used herein, is the pharmacokinetic profile measured after the initial dose of meloxicam. The compositions can be formulated in any way as described below.

A preferred nanoparticulate meloxicam formulation of the invention exhibits in comparative pharmacokinetic testing with a standard commercial formulation of meloxicam, such as MOBIC® from Boehringer Ingelheim Pharmaceuticals, Inc., a Tₘₐₓ not greater than about 90%, not greater than about 80%, not greater than about 70%, not greater than about 60%, not greater than about 50%, not greater than about 30%, not greater than about 25%, not greater than about 20%, not greater than about 15%, or not greater than about 10% of the Tₘₐₓ exhibited by a standard commercial meloxicam formulation, *e.g.*, MOBIC® tablets.

In addition, a preferred nanoparticulate meloxicam formulation of the invention exhibits in comparative pharmacokinetic testing with a standard commercial formulation of meloxicam, such as MOBIC® from Boehringer Ingelheim Pharmaceuticals, Inc., a Cₘₐₓ which is greater than about 20%, greater than about 40%, greater than about 60%, greater than about 80%, greater than about 100%, greater than about 140%, greater than about 180%, greater than about 200%, greater than about 240%, greater than about 280%, greater than about 300%, greater than about 340%, greater than about 380%, or greater than about 400% of the Cₘₐₓ exhibited by a standard commercial meloxicam formulation, e.g., MOBIC® capsules.

Any nanoparticulate meloxicam formulation giving the desired pharmacokinetic profile is suitable for administration according to the present methods.

### 1. Meloxicam Particles

As used herein the term meloxicam, which is the active ingredient in the composition, is used to mean meloxicam (4-hydroxy-2-methyl-N-(5-methyl-2-thiazolyl)-2-H-1,2-benzothiazine-3-carboxamide 1,1-dioxide) or any salt thereof. Meloxicam can be present in a crystalline phase, an amorphous phase, a semi-crystalline phase, a semi-amorphous phase, or a mixture thereof.

Nanoparticulate meloxicam compositions are contemplated to be useful in treatment and/or prevention of a wide range of conditions and disorders mediated by COX-2, including but not limited to, disorders characterized by inflammation, pain, and/or fever. Such compositions are especially useful as anti-inflammatory agents, such as in treatment of arthritis, with the additional benefit of having significantly less harmful side effects than compositions of conventional NSAIDs that lack selectivity for COX-2 over COX-1. In particular, such compositions have reduced potential for gastrointestinal toxicity and gastrointestinal irritation including upper gastrointestinal ulceration and bleeding, reduced potential' for renal side effects such as reduction in renal function leading to fluid retention and exacerbation of hypertension, reduced effect on bleeding times including inhibition of platelet function, and possibly a lessened ability to induce asthma attacks in aspirin-sensitive asthmatic subjects, by comparison with compositions of conventional NSAIDs.

Thus, nanoparticulate meloxicam compositions of the invention are particularly useful as an alternative to conventional NSAIDs where such NSAIDs are contraindicated, for example in patients with peptic ulcers, gastritis, regional enteritis, ulcerative colitis, diverticulitis, or with a recurrent history of gastrointestinal lesions; gastrointestinal bleeding; coagulation disorders including anemia such as hypoprothrombinemia, hemophilia, or other bleeding problems; kidney disease; or in patients prior to surgery or patients taking anticoagulants.

Because of the rapid onset of therapeutic effect observed with the compositions of the invention, these compositions have particular advantages over prior conventional formulations for treatment of acute COX-2 mediated disorders, especially for relief of pain, for example in headache, including sinus headache and migraine.

Meloxicam is also useful in treating and/or preventing, for example, arthritic disorders, gastrointestinal conditions, inflammatory conditions, pulmonary inflammation, opthalmic diseases, central nervous systems disorders, pain, inflammation-related cardiovascular disorders, angiogenesis-related disorders, benign and malignant tumors, adenomatous polyps, disorders of the female reproductive system such as endometriosis, osteoporosis, dysmenorrhea, premature labor, asthma, eosinophil-related disorders, pyrexia, bone resorption, nephrotoxicity, hypotension, arthrosis, joint stiffness, kidney disease, liver disease including hepatitis, acute mastitis, diarrhea, colonic adenomas, bronchitis, allergic neuritis, cytomegalovirus infectivity, apoptosis including HIV-induced apoptosis, lumbago; skin-related conditions such as psoriasis, eczema, acne, burns, dermatitis, and ultraviolet radiation damage including sunburn; allergic rhinitis, respiratory distress syndrome, and endotoxin shock syndrome. Nanoparticulate meloxicam is also useful as an immunosuppressive agent.

Exemplary forms of arthritic disorders which can be treated include, but are not limited to, osteoarthritis, rheumatoid arthritis, spondyloarthropathies, gouty arthritis, juvenile arthritis, gout, ankylosing spondylitis, systemic lupus erythematosus, bursitis, tendinitis, myofascial pain, carpal tunnel syndrome, fibromyalgia syndrome, infectious arthritis, psoriatic arthritis, reiter's syndrome, and scleroderma

Exemplary gastrointestinal conditions or ulcerative diseases which can be treated include, but are not limited to, inflammatory bowel disease, Crohn's disease, gastritis, irritable bowel syndrome, ulcerative colitis, gastric ulcer, pathological but non-malignant conditions such as hemangiomas, including infantile hemaginomas, angiofibroma of the nasopharynx, and avascular necrosis of bone.

Exemplary inflammation conditions which can be treated include, but are not limited to, migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury including brain edema, myocardial ischemia, post-operative inflammation including that following ophthalmic surgery such as cataract surgery or refractive surgery, and the like.

Exemplary pulmonary inflammation conditions which can be treated include, but are not limited to, inflammation associated with viral infections and cystic fibrosis, and in bone resorption such as that associated with osteoporosis.

Exemplary opthalmic diseases or conditions which can be treated include, but are not limited to, retinitis, conjunctivitis, retinopathies, uveitis, ocular photophobia, acute injury to the eye tissue, corneal graft rejection, ocular neovascularization, retinal neovascularization including neovascularization following injury or infection, diabetic retinopathy, macular degeneration, retrolental fibroplasia, glaucoma, and neovascular glaucoma.

Exemplary central nervous system disorders which can be treated include, but are not limited to, cortical dementias including Alzheimer's disease, neurodegeneration, and central nervous system damage resulting from stroke, ischemia, and trauma. The term "treatment" in the present context includes partial or total inhibition of dementias, including Alzheimer's disease, vascular dementia, multi-infarct dementia, pre-senile dementia, alcoholic dementia, and senile dementia.

Exemplary pain conditions which can be treated include, but are not limited to, postoperative pain, pain resulting from battle field wounds, dental pain, muscular pain, pain resulting from cancer, headaches, including sinus headache and migraine, menstrual cramps, and pain associated with inflammation.

The compositions of the invention are useful for relief of pain, fever, and inflammation in a variety of conditions including rheumatic fever, influenza, and other viral infections including common cold, low back, and neck pain, dysmenorrhea, headache, toothache, sprains and strains, myositis, neuralgia, synovitis, arthritis, including rheumatoid arthritis, degenerative joint diseases (osteoarthritis), gout, ankylosing spondylitis, bursitis, burns, and trauma following surgical and dental procedures.

Exemplary inflammation-related cardiovascular disorders which can be treated or prevented using the compositions of the invention include, but are not limited to, vascular diseases, coronary artery disease, aneurysm, vascular rejection, arteriosclerosis, atherosclerosis including cardiac transplant atherosclerosis, myocardial infarction, embolism, stroke, thrombosis including venous thrombosis, angina including unstable angina, coronary plaque inflammation, bacterial-induced inflammation including Chlamydia-induced inflammation, viral induced inflammation, and inflammation associated with surgical procedures such as vascular grafting including coronary artery bypass surgery, revascularization procedures including angioplasty, stent placement, endarterectomy, or other invasive procedures involving arteries, veins, and capillaries.

Exemplary angiogenesis-related disorders include, but are not limited to, inhibition of tumor angiogenesis. Such compositions are useful in treatment of neoplasia, including metastasis, benign and malignant tumors, and neoplasia including cancer, such as colorectal cancer, brain cancer, bone cancer, epithelial cell-derived neoplasia (epithelial carcinoma) such as basal cell carcinoma, adenocarcinoma, gastrointestinal cancer such as lip cancer, mouth cancer, esophageal cancer, small bowel cancer, stomach cancer, colon cancer, liver cancer, bladder cancer, pancreas cancer, ovary cancer, cervical cancer, lung cancer, breast cancer, skin cancer such as squamous cell and basal cell cancers, prostate cancer, renal cell carcinoma, and other known cancers that effect epithelial cells throughout the body. Neoplasias for which compositions of the invention are contemplated to be particularly useful are gastrointestinal cancer, Barrett's esophagus, liver cancer, bladder cancer, pancreatic cancer, ovarian cancer, prostate cancer, cervical cancer, lung cancer, breast cancer and skin cancer. The nanoparticulate meloxicam compositions of the invention can also be used to treat fibrosis that occurs with radiation therapy.

The compositions of the invention can be used to treat subjects having adenomatous polyps, including those with familial adenomatous polyposis (FAP). Additionally, such compositions can be used to prevent polyps from forming in patients at risk ofFAP.

Because the meloxicam compositions of the invention inhibit prostanoid-induced smooth muscle contraction by inhibiting synthesis of contractile prostanoids, the compositions can be used in the treatment of dysmenorrhea, premature labor, asthma, and eosinophil-related disorders.

The compositions of the invention are also useful in treating indications where anti-inflammatory agents, anti-angiogenesis agents, antitumorigenic agents, immunosuppressive agents, NSAIDs, COX-2 inhibitors, analgesic agents, anti-thrombotic agents, narcotics, or antifebrile agents are typically used.

### 2. Non-Meloxicam Active Agents

The nanoparticulate meloxicam compositions of the invention can additionally comprise one or more non-meloxicam active agents, in either a conventional or nanoparticulate form. The non-meloxicam active agents can be present in a crystalline phase, an amorphous phase, a semi-crystalline phase, a semi-amorphous phase, or a mixture thereof.

If the non-meloxicam active agent is in a nanoparticulate form, then it will have one or more surface stabilizers adsorbed on or associated with the surface of the active agent. In addition, if the active agent is in a nanoparticulate form it is preferably poorly soluble, and is dispersible in at least one liquid dispersion medium. By "poorly soluble" it is meant that the active agent has a solubility in a liquid dispersion medium of less than about 10 mg/mL, and preferably less than about 1 mg/mL. Useful liquid dispersion mediums include, but are not limited to, water, aqueous salt solutions, safflower oil, and solvents such as ethanol, t-butanol, hexane, and glycol.

Such active agents can be, for example, an active, therapeutic, or diagnostic agent. A therapeutic agent can be a pharmaceutical agent, including biologics such as proteins, peptides, and nucleotides, or a diagnostic agent, such as a contrast agent, including x-ray contrast agents. The active agent can be selected from a variety of known classes of drugs, including, for example, proteins, peptides, nucleotides, anti-obesity drugs, nutraceuticals, dietary supplements, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, parasympathomimetics, parathyroid calcitonin and biphosphonates, prostaglandins, radiopharmaceuticals, sex hormones (including steroids), anti-allergic agents, stimulants and anoretics, sympathomimetics, thyroid agents, vasodilators, and xanthines.

A description of these classes of active agents and a listing of species within each class can be found in Martindale's The Extra Pharmacopoeia, 31st Edition (The Pharmaceutical Press, London, 1996), specifically incorporated by reference. The active agents are commercially available and/or can be prepared by techniques known in the art.

Exemplary nutraceuticals and dietary supplements are disclosed, for example, in Roberts et al., Nutraceuticals: The Complete Encyclopedia of Supplements, Herbs, Vitamins, and Healing Foods (American Nutraceutical Association, 2001), which is specifically incorporated by reference. A nutraceutical or dietary supplement, also known as phytochemicals or functional foods, is generally any one of a class of dietary supplements, vitamins, minerals, herbs, or healing foods that have medical or pharmaceutical effects on the body. Exemplary nutraceuticals or dietary supplements include, but are not limited to, lutein, folic acid, fatty acids (*e.g.,* DHA and ARA), fruit and vegetable extracts, vitamin and mineral supplements, phosphatidylserine, lipoic acid, melatonin, glucosamine/chondroitin, Aloe Vera, Guggul, glutamine, amino acids (*e.g.,* iso-leucine, leucine, lysine, methionine, phenylanine, threonine, tryptophan, and valine), green tea, lycopene, whole foods, food additives, herbs, phytonutrients, antioxidants, flavonoid constituents of fruits, evening primrose oil, flax seeds, fish and marine animal oils, and probiotics. Nutraceuticals and dietary supplements also include bio-engineered foods genetically engineered to have a desired property, also known as "pharmafoods."

Nanoparticulate meloxican compositions useful in methods of the present invention can be used in combination therapies with opioids and other analgesics, including narcotic analgesics, Mu receptor antagonists, Kappa receptor antagonists, non-narcotic (i.e., non-addictive) analgesics, monoamine uptake inhibitors, adenosine regulating agents, cannabinoid derivatives, Substance P antagonists, neurokinin-1 receptor antagonists and sodium channel blockers, among others.

Preferred combination therapies comprise a composition useful in methods of the invention with one or more compounds selected from aceclofenac, acemetacin, e-acetamidocaproic acid, acetaminophen, acetaminosalol, acetanilide, acetylsalicylic acid (aspirin), S-adenosylmethionine, alclofenac, alfentanil, allylprodine, alminoprofen, aloxiprin, alphaprodine, aluminum bis(acetylsalicylate), amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, bendazac, benorylate, benoxaprofen, benzpiperylon, benzydamine, benzylmorphine, bermoprofen, bezitramide, α-bisabolol, bromfenac, p-bromoacetanilide, 5-bromosalicylic acid acetate, bromosaligenin, bucetin, bucloxic acid, bucolome, bufexamac, bumadizon, buprenorphine, butacetin, butibufen, butophanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac sodium, difenamizole, difenpiramide, diflunisal, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, p-lactophenetide, lefetamine, levorphanol, lofentanil, lonazolac, lomoxicam, loxoprofen, lysine acetylsalicylate, magnesium acetylsalicylate, meclofenamic acid, mefenamic acid, meperidine, meptazinol, mesalamine, metazocine, methadone hydrochloride, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paranyline, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamide, salicin, salicylamide, salicylamide o-acetic acid, salicylsulfuric acid, salsalte, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, viminol, xenbucin, ximoprofen, zaltoprofen, and zomepirac. *See* The Merck Index, 12th Edition (1996), Therapeutic Category and Biological Activity Index, lists therein headed "Analgesic", "Anti-inflammatory", and "Antipyretic"). Particularly preferred combination therapies comprise use of a nanoparticulate meloxicam composition of the invention with an opioid compound, more particularly where the opioid compound is codeine, meperidine, morphine, or a derivative thereof.

The compound to be administered in combination with a nanoparticulate meloxicam composition of the invention can be formulated separately from said composition or co-formulated with said composition. Where a meloxicam composition is co-formulated with a second drug, for example an opioid drug, the second drug can be formulated in immediate-release, rapid-onset, sustained-release, or dual-release form.

In an embodiment of the invention, particularly where the COX-2 mediated condition is headache or migraine, the nanoparticulate meloxicam composition is administered in combination therapy with a vasomodulator, preferably a xanthine derivative having vasomodulatory effect, more preferably an alkylxanthine compound.

Combination therapies wherein an alkylxanthine compound is co-administered with a nanoparticulate meloxicam composition as provided herein are embraced by the present embodiment of the invention whether or not the alkylxanthine is a vasomodulator and whether or not the therapeutic effectiveness of the combination is to any degree attributable to a vasomodulatory effect. The term "alkylxanthine" herein embraces xanthine derivatives having one or more C₁₋₄ alkyl substituents, preferably methyl, and pharmaceutically acceptable salts of such xanthine derivatives. Dimethylxanthines and trimethylxanthines, including caffeine, theobromine, and theophylline, are especially preferred. Most preferably, the alkylxanthine compound is caffeine.

Exemplary COX-2 inhibitors which can be formulated in combination with the nanoparticulate meloxicam composition of the invention include, but are not limited to, celecoxib, rofecoxib (Vioxx^{®}), meloxicam (MOBIC^{®}, co-marketed by Abbott Laboratories, Chicago, IL, and Boehringer Ingelheim Pharmaceuticals, Inc.), valdecoxib (G.D. Searle & Co.), parecoxib (G.D. Searle & Co.), MK-966 (Merck, in Phase III studies), etoricoxib (MK-663; Merck, in Phase II studies), SC-236 (chemical name of 4-[5-(4-chlorophenyl)-3-(trifluoromethyl)-1H-pyrazol-1-yl)] benzenesulfonamide; G.D. Searle & Co., Skokie, IL); NS-398 (N-(2-cyclohexyloxy-4-nitrophenyl)methane sulfonamide; Taisho Pharmaceutical Co., Ltd., Japan); SC-58125 (methyl sulfone spiro(2.4)hept-5-ene I; Pharmacia/Searle & Co.); SC-57666 (Pharmacia/Searle & Co.); SC-58635 (celexcoxib; Pharmacia/Searle & Co.); SC-558 (Pharmacia/Searle & Co.); SC-560 (Pharmacia/Searle & Co.); etodolac (Lodine^{®}, Wyeth-Ayerst Laboratories, Inc.); DFU (5,5-dimethyl-3-(3-fluorophenyl)-4-(4-methylsulfonyl)phenyl 2(5H)-furanone); MK-476, L-745337, L-761066, L-761000, L-748780, and L-748731 (all Merck & Co.); DUP-697 (5-Bromo-2-(4-fluorophenyl)-3-(4-(methylsulfonyl)phenyl; DuPont Merck Pharmaceutical Co.); PGV 20229 (1-(7-tert.-butyl-2,3-dihydro-3,3-dimethylbenzo(b)furan-5-yl)-4-cyclopropylbutan-1-one) (Procter & Gamble Pharmaceuticals); T-614 (3-formylamino-7-methylsulfonylamino-6-phenoxy-4H-1-benzopyran-4-one; Toyama Corp., Japan); BF 389 (Biofor, USA); PD 136005, PD 142893, and PD 145065 (all Parke-Davis/Warner-Lambert Co.); flurbiprofen (Ansaid®; Pharmacia & Upjohn); nimesulide (N1M-03, Mesulid®; Hisamitsu, Japan); nabumetone (Relafen^{®}; SmithKline Beecham, plc); flosulide (CGP 28238; Novartis/Ciba Geigy); piroxicam (Feldene^{®}; Pfizer); dicofenac (Voltaren^{®} and Cataflam^{®}, Novartis); COX-189 (Novartis); D 1367 (Celltech Chiroscience, plc); R 805 (4 nitro 2 phenoxymethane sulfonanilide); R 807 (3 benzoyldifluoromethane sulfonanilide, diflumidone); JTE-522 (Japan Tobacco, Japan); FK-3311 (4'-Acetyl-2'-(2,4-difluorophenoxy)methanesulfonanilide; Fujisawa, Japan); FK 867 (Fujisawa, Japan); FR 115068 (Fujisawa, Japan); GR 253035 (Glaxo Wellcome); RWJ 63556 (Johnson & Johnson); RWJ 20485 (Johnson & Johnson); ZK 38997 (Schering); S 2474 ((E)-(5)-(3,5-di-tert-butyl-4-hydroxybenzylidene)-2-ethyl-1,2-isothiazolidine-1,1 -dioxide indomethacin; Shionogi & Co., Ltd., Japan); CL 1004 (Parke-Davis); RS 57067 (Hoffmann La Roche); RS 104894 (Hoffmann La Roche); SC 41930 (Monsanto); SB 205312 (SmithKline Beecham); SKB 209670 (SmithKline Beecham, plc); and Ono 1078 (Ono Pharmaceutical Co., Japan).

### 3. Surface Stabilizers

The choice of one or more surface stabilizers for meloxicam is non-trivial and required extensive experimentation to realize a desirable formulation. Accordingly, the present invention is directed to the surprising discovery that nanoparticulate meloxicam compositions can be made.

Combinations of more than one surface stabilizer can be used in the invention. Useful surface stabilizers which can be employed in the invention include, but are not limited to, known organic and inorganic pharmaceutical excipients. Such excipients include various polymers, low molecular weight oligomers, natural products, and surfactants. Preferred surface stabilizers include nonionic, cationic, and ionic surfactants.

Representative examples of surface stabilizers include hydroxypropyl methylcellulose, hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (e.g., macrogol ethers such as cetomacrogol 1000), polyoxyethylene castor oil derivatives, polyoxyethylene sorbitan fatty acid esters (e.g., the commercially available Tweens^{®} such as *e.g.*, Tween 20^{®} and Tween 80^{®} (ICI Speciality Chemicals)); polyethylene glycols (*e.*g., Carbowaxs 3550^{®} and 934^{®} (Union Carbide)), polyoxyethylene stearates, colloidal silicon dioxide, phosphates, carboxymethylcellulose calcium, carboxymethylcellulose sodium, methylcellulose, hydroxyethylcellulose, hydroxypropylmethylcellulose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (e.g., Pluronics F68^{®} and F108^{®}, which are block copolymers of ethylene oxide and propylene oxide); poloxamines (e.g., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508, a poloxamine) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-lOG^{®} or Surfactant 10-G^{®} (Olin Chemicals, Stamford, CT); Crodestas SL-40^{®} (Croda, Inc.); and SA9OHCO, which is C₁₈H₃₇CH₂C(O)N(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

Examples of useful cationic surface stabilizers include, but are not limited to, polymers, biopolymers, polysaccharides, cellulosics, alginates, phospholipids, and nonpolymeric compounds,-such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336^{™}), POLYQUAT 10^{™} (polyquaternium 10; Buckman Laboratories, TN), tetrabutylammonium bromide, benzyl trimethylammonium bromide, choline esters (such as choline esters of fatty acids), benzalkonium chloride, stearalkonium chloride compounds (such as stearyltrimonium chloride and Di-stearyldimonium chloride), cetyl pyridinium bromide or chloride, halide salts of quaternized polyoxyethylalkylamines, MIRAPOL^{™} (quaternized ammonium salt polymers) and ALKAQUAT^{™} (benzalkonium chloride) (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric surface stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄. comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quatemium-15), distearyldimonium chloride (Quatemium-5), dodecyl dimethyl ethylbenzyl ammonium chloride(Quaternium-14), Quatemium-22, Quatemium-26, Quatemium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquatemium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated by reference.

The surface stabilizers are commercially available and/or can be prepared by techniques known in the art.

### 4. Other Pharmaceutical Excipients

Pharmaceutical compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents are lactose monohydrate, lactose anhydrous, and various starches; examples of binding agents are various celluloses and cross-linked polyvinylpyrrolidone, microcrystalline cellulose, such as Avicel® PH101 and Avicel® PH102, microcrystalline cellulose, and silicified microcrystalline cellulose (ProSolv SMCC^{™}).

Suitable lubricants, including agents that act on the flowability of a powder to be compressed, are colloidal silicon dioxide, such as Aerosil^{®} 200, talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, mint flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### 5. Nanoparticulate Meloxicam and Active Agent Particle Size

The compositions of the invention comprise meloxicam nanoparticles which have an effective average particle size of less than about 2000 nm (i.e., 2 microns), less than about 1500 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

If the composition additionally comprises one or more non-meloxicam nanoparticulate active agents, then such active agents have an effective average particle size of less than about 2000 nm (*i.e.,* 2 microns), less than about 1500 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

By "an effective average particle size of less than about 2000 nm" it is meant that at least 50% of the meloxicam or nanoparticulate active agent particles have a particle size of less than about 2000 nm when measured by the above-noted techniques. Preferably, at least 70%, 90%, or 95% of the meloxicam particles have a particle size of less than the desired particle size, *e.g.,* 2000 nm, 1500 nm, 1000 nm, *etc.*

### 6. Concentration of Nanoparticulate Meloxicam,

### Surface Stabilizers, and Optional One or More Active Agents

The relative amounts of meloxicam and one or more surface stabilizers can vary widely. The optimal amount of the individual components can depend, for example, upon the particular active agent selected, the hydrophilic lipophilic balance (HLB), melting point, and the surface tension of water solutions of the stabilizer, etc.

The concentration of meloxicam can vary from about 99.5% to about 0.001%, from about 95% to about 0.1 %, and from about 90% to about 0.5%, by weight, based on the total combined weight of the meloxicam and at least one surface stabilizer, not including other excipients.

The concentration of the at least one surface stabilizer can vary from about 0.01% to about 99.5%, from about 0.1% to about 95%, and from about 0.5% to about 90%, by weight, based on the total combined dry weight of the meloxicam and at least one surface stabilizer, not including other excipients.

### B. Methods of Making Nanoparticulate Formulations

The nanoparticulate meloxicam compositions can be made using, for example, milling, homogenization, or precipitation techniques. Exemplary methods of making nanoparticulate compositions are described in the'684 patent. Methods of making nanoparticulate compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation of Nanoparticulate Pharmaceutical Agents with Crystal Growth Modifiers;" U.S. Patent No. 5,662,883 for "Co-Microprecipitation of Nanoparticulate Pharmaceutical Agents with Crystal Growth Modifiers;" U.S. Patent No. 5,560,932 for "Microprecipitation of Nanoparticulate Pharmaceutical Agents;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation," all of which are specifically incorporated by reference.

One or more non-meloxicam active agents can also be reduced in size at the same time as meloxicam, to produce a nanoparticulate meloxican and nanoparticulate non-meloxicam active agent composition. A non-meloxicam active agent, which is either conventional or nanoparticulate sized, can also be added to the nanoparticulate meloxicam composition after size reduction.

In yet another embodiment of the invention, nanoparticulate meloxicam compositions of the invention can be made in which the formulation comprises multiple nanoparticulate meloxicam compositions, each of which has a different effective average particle size. Such a composition can be made by preparing the individual nanoparticulate meloxicam formulations using, for example, milling, precipitation, or homogenization techniques, followed by combining the different compositions to prepare a single dosage form.

### 1. Milling to Obtain Nanoparticulate Meloxicam Dispersions

Milling meloxicam to obtain a nanoparticulate dispersion comprises dispersing meloxicam particles in a liquid dispersion medium in which meloxicam is poorly soluble, followed by applying mechanical means in the presence of rigid grinding media to reduce the particle size of meloxicam to the desired effective average particle size. The dispersion medium can be, for example, water, safflower oil, ethanol, t-butanol, glycerin, polyethylene glycol (PEG), hexane, or glycol.

The meloxicam particles can be reduced in size in the presence of at least one surface stabilizer. Alternatively, the meloxicam particles can be contacted with one or more surface stabilizers after attrition. Other compounds, such as a diluent, can be added to the meloxicam/surface stabilizer composition during the size reduction process. Dispersions can be manufactured continuously or in a batch mode. The resultant nanoparticulate meloxicam dispersion can be utilized in solid or liquid dosage formulations, such as liquid dispersions, gels, aerosols, ointments, creams, controlled release formulations, fast melt formulations, lyophilized formulations, tablets, capsules, delayed release formulations, extended release formulations, pulsatile release formulations, mixed immediate release and controlled release formulations, *etc*.

### 2. Precipitation to Obtain Nanoparticulate Meloxicam Compositions

Another method of forming the desired nanoparticulate meloxicam composition is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method comprises, for example: (1) dissolving meloxicam in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means. The resultant nanoparticulate meloxicam dispersion can be utilized in solid or liquid dosage formulations, such as liquid dispersions, gels, aerosols, ointments, creams, controlled release formulations, fast melt formulations, lyophilized formulations, tablets, capsules, delayed release formulations, extended release formulations, pulsatile release formulations, mixed immediate release and controlled release formulations, etc.

### 3. Homogenization to Obtain Meloxicam

### Nanoparticulate Compositions

Exemplary homogenization methods of preparing active agent nanoparticulate compositions are described in U.S. Patent No. 5,510,118, for "Process of Preparing Therapeutic Compositions Containing Nanoparticles." Such a method comprises dispersing meloxicam particles in a liquid dispersion medium, followed by subjecting the dispersion to homogenization to reduce the particle size of the meloxicam to the desired effective average particle size. The meloxicam particles can be reduced in size in the presence of at least one surface stabilizer. Alternatively, the meloxicam particles can be contacted with one or more surface stabilizers either before or after attrition. Other compounds, such as a diluent, can be added to the meloxicam/surface stabilizer composition either before, during, or after the size reduction process. Dispersions can be manufactured continuously or in a batch mode. The resultant nanoparticulate meloxicam dispersion can be utilized in solid or liquid dosage formulations, such as liquid dispersions, gels, aerosols, ointments, creams, controlled release formulations, fast melt formulations, lyophilized formulations, tablets, capsules, delayed release formulations, extended release formulations, pulsatile release formulations, mixed immediate release and controlled release formulations, etc.

### C. Methods of Using Meloxicam Formulations of the Current Invention

The meloxicam compositions of the present invention can be administered to a subject via any conventional means including, but not limited to, orally, rectally, ocularly, parenterally (e.g., intravenous, intramuscular, or subcutaneous), intracisternally, pulmonary, intravaginally, intraperitoneally, locally (e.g., powders, ointments or drops), or as a buccal or nasal spray. As used herein, the term "subject" is used to mean an animal, preferably a mammal, including a human or non-human. The terms patient and subject may be used interchangeably.

The present invention provides a method of rapidly increasing the plasma levels of meloxicam in a subject. Such a method comprises administering to a subject an effective amount of a composition comprising nanoparticulate meloxicam.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles including water, ethanol, polyols (propyleneglycol, polyethylene-glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

The nanoparticulate compositions may also contain adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

Solid dosage forms for oral administration include, but are not limited to, capsules, tablets, pills, powders, and granules. In such solid dosage forms, the active agent is admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to the active agent, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

Ocular dosage forms of the nanoparticulate meloxicam of the invention preferably do not include cross-linked carboxyl-containing polymers, used as excipients, as described in U.S. Pat. No. 5,192,535. Such excipients can be undesirable.

One of ordinary skill will appreciate that effective amounts of meloxicam can be determined empirically and can be employed in pure form or, where such forms exist, in pharmaceutically acceptable salt, ester, or prodrug form. Actual dosage levels of meloxicam in the nanoparticulate compositions of the invention may be varied to obtain an amount of meloxicam that is effective to obtain a desired therapeutic response for a particular composition and method of administration. The selected dosage level therefore depends upon the desired therapeutic effect, the route of administration, the potency of the administered meloxicam, the desired duration of treatment, and other factors.

Dosage unit compositions may contain such amounts of such submultiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration, and rate of excretion of the agent; the duration of the treatment; drugs used in combination or coincidental with the specific agent; and like factors well known in the medical arts.

### 1. Use of Additional Active Agents

The methods of the invention also encompass administering a nanoparticulate meloxicam composition of the invention in combination with one or more non-meloxicam active agents, in either a conventional or nanoparticulate form. In general, such additional active agents do not include vasomodulators, such as those described in U.S. Published Application No. 2002007328.

### 2. Treatment Applications

The compositions of the invention are useful in treating and/or preventing, for example, conditions in which NSAIDs are contraindicated, arthritic disorders, gastrointestinal conditions, inflammatory conditions, pulmonary inflammation, opthalmic diseases, central nervous systems disorders, pain, fever, inflammation-related cardiovascular disorders, angiogenesis-related disorders, benign and malignant tumors, adenomatous polyps, fibrosis which occurs with radiation treatment, disorders of the female reproductive system such as endometriosis, osteoporosis, dysmenorrhea, premature labor, asthma, eosinophil-related disorders, pyrexia, bone resorption, nephrotoxicity, hypotension, arthrosis, joint stiffness, kidney disease, liver disease including hepatitis, acute mastitis, diarrhea, colonic adenomas, bronchitis, allergic neuritis, cytomegalovirus infectivity, apoptosis including HIV-induced apoptosis, lumbago, skin-related conditions such as psoriasis, eczema, acne, bums, dermatitis, and ultraviolet radiation damage including sunburn, allergic rhinitis, respiratory distress syndrome, and endotoxin shock syndrome. Nanoparticulate meloxicam is also useful as an immunosuppressive agent.

The compositions of the invention are also useful in treating indications where anti-inflammatory agents, anti-angiogenesis agents, antitumorigenic agents, immunosuppressive agents, NSAIDs, COX-2 inhibitors, analgesic agents, anti-thrombotic agents, narcotics, or antifebrile agents are typically used.

More detailed descriptions of the arthritis disease conditions osteoarthritis and rheumatoid arthritis are given below.

### 3. Arthritis

Although the term "arthritis" literally means joint inflammation, arthritis refers to a group of more than 100 rheumatic diseases and conditions that can cause pain, stiffness, and swelling in the joints. Certain conditions may affect other parts of the body, such as the muscles, bones, and some internal organs, and can result in debilitating, and sometimes life-threatening, complications. If left undiagnosed and untreated, arthritis can cause irreversible damage to the joints.

Arthritis already affects more than 42 million Americans in its chronic form, including 300,000 children. By 2020, the Center for Disease Control estimates that 60 million people will be affected, and that more than 11 million will be disabled. *See* http://www.fda.gov/fdac/features/2000/300_arth.html. The two most common forms of the disease, osteoarthritis and rheumatoid arthritis, have the greatest public health implications, according to the Arthritis Foundation. *Id.* Other common forms arthritis and related conditions include juvenile arthritis, gout, ankylosing spondylitis, systemic lupus erythematosus, bursitis, tendinitis and myofascial pain, carpal tunnel syndrome, fibromyalgia syndrome, infectious arthritis, psoriatic arthritis, reiter's syndrome, and scleroderma.

Osteoarthritis, previously known as "degenerative joint disease," results from wear and tear on the joints, like the knees, hips and fingers, or from an injury. The pressure of gravity causes physical damage to the joints and surrounding tissues, leading to pain, tenderness, swelling, and decreased function. Initially, osteoarthritis is non-inflammatory and its onset is subtle and gradual, usually involving one or only a few joints. Pain is the earliest symptom, usually made worse by repetitive use. Risk factors for osteoarthritis include aging, joint trauma, obesity, repetitive joint use, and diabetes.

Many people may have only mild discomfort with osteoarthritis. But as cartilage covering the ends of bones in the joints wears away, considerable pain, inflammation, and loss of movement can result. Osteoarthritis affects all parts of a joint, causing pain and stiffness, especially after physical activity.

Osteoarthritis affects more than 20 million people, and the risk of getting it increases with age. It is the most common joint disease. About one-third of people over age 35 show some signs of osteoarthritis when they have x-rays. The joints most often affected are the knee, hip, and hand. In people over age 55, osteoarthritis of the hip is more common in men and osteoarthritis of the fingers is more common in women. Osteoarthritis of the knees causes problems for both men and women. In the United States, about 100,000 people can't walk from their beds to their bathrooms without help because of osteoarthritis of the knee or hip.

Rheumatoid arthritis is an autoimmune disease that occurs when the body's own immune system mistakenly attacks the synovium (cell lining inside the joint). This chronic, potentially disabling disease causes pain, stiffness, swelling, and loss of function in the joints. Rheumatoid arthritis affects about one in every 100 people. It affects women three times more often than men. The people most likely to develop rheumatoid arthritis are those between the ages of 35 and 50.

Rheumatoid arthritis affects the joint membranes, cartilage, and bones the way osteoarthritis does. But unlike osteoarthritis, rheumatoid arthritis can also affect the whole body, with loss of appetite, a general feeling of being unwell, and other symptoms. The exact cause of rheumatoid arthritis is unknown, although it may be triggered by the immune system (the body's defenses) turning against the body.

The following examples are given to illustrate the present invention. It should be understood, however, that the invention is not to be limited to the specific conditions or details described in these examples. Throughout the specification, any and all references to a publicly available document, including a U.S. patent, are specifically incorporated by reference.

### Example 1

The purpose of this example is describe how a nanoparticle dispersion of meloxicam could be prepared.

A desired quantity of meloxicam and at least one surface stabilizer can be milled in the presence of suitable rigid grinding media for a suitable period of time in, for example, a DYNO®-Mill KDL (Willy A. Bachofen AG, Maschinenfabrik, Basel, Switzerland), a roller mill (U.S. Stoneware), or a NanoMill® (Elan Drug Delivery Inc.) (*see e.g.,* WO 00/72973 for "Small-Scale Mill and Method Thereof").

The mean particle size of the resultant compositions, as measured using, for example, a Horiba LA-910 Laser Scattering Particle Size Distribution Analyzer (Horiba Instruments, Irvine, CA) is expected to be less than 2 microns. The dispersion is expected to exhibit excellent stability over an extended period of time over a range of temperatures.

### Example 2

The purpose of this example is to describe how a solid dose form of nanoparticulate meloxicam could be prepared.

The nanoparticulate dispersion of Example 1 can be spray dried, lyophilized, or spray granulated to form a powder. The resulting powder or granules of nanoparticulate meloxicam can then be mixed with the suitable excipients, such as the ingredients listed in Table 1, followed by tableting.

| **TABLE 1** | |
|---|---|
| **Ingredient Description** | **Percent of Total (Per Tablet)** |
| Nanoparticulate Meloxicam Spray Dried Powder | 50.2 |
| Pregelatinized Starch NF (Colorcon® Starch 1500) | 20.0 |
| Microcrystalline Cellulose NF (Avicel® PH101) | 20.0 |
| Sodium Starch Glycorlate (Explotab®) | 5.3 |
| Croscarmellose Sodium USP (Ac-Di-Sol®) | 4.0 |
| Magnesium Stearate NF | 0.5 |
| Totals | 100.0 |

The tablets are expected to show excellent redispersion in water as well as in simulated biological fluids. This is significant as redispersion in simulated biological fluids is predictive of redispersion under *in vivo* conditions.

### Example 3

The purpose of this example was to prepare nanoparticulate dispersions of meloxicam stabilized with various surface modifiers intended for injectable administration.

Aqueous dispersions of 5 wt. % meloxicam (Unichem Laboratories, Ltd.) and 1 wt. % stabilizer (see Table 2, below) were charged into a NanoMill® milling system (Elan Drug Delivery, Inc., King of Prussia, PA; *see e.g.,* U.S. Patent No. 6,431,478 for "Small Scale Mill") equipped with a 10cc batch chamber.

The following NanoMill® milling system parameters were used for all of the formulations: mill speed = 5500 rpm; total milling time = 1 hour; polymeric milling media type = PolyMill^{™}-200 (The Dow Chemical Co.); and a media load sufficient to process.

Particle size analysis of the resultant milled dispersions was performed using a Horiba LA-910 particle size analyzer ((Horiba Instruments, Irvine, CA). The results are shown below in Table 2. In the table below, the value for D50 is the particle size below which 50% of the active agent particles fall. Similarly, D90 is the particle size below which 90% of the active agent particles fall.

| **TABLE 2** | | | | | |
|---|---|---|---|---|---|
| **Stabilizer** | **Stabilizer Manufacturer** | **Mean (nm)** | **D50 (nm)** | **D90 (nm)** | **Optical Microscopy*** |
| Pluronic^{®} F68 (poloxamer 188) | BASF | 133 | 110 | 226 | Stable |
| Pluronic^{®} F108 (poloxamer 388) | BASF | 129 | 108 | 219 | Stable |
| Kollidon^{®} 12 PF | BASF | 98 | 90 | 125 | Stable |
| Kollidon^{®} 17 PF | BASF | 98 | 95 | 135 | Stable |
| Polysorbate 80 | Spectrum | 227 | 227 | 322 | Stable |
| Sodium Deoxycholate | Prodotti Chimici | 119 | 101 | 198 | Stable |
| Lecithin | Penta | 190 | 169 | 271 | Mild aggregation at initial |
| Lysozyme | Fordras | 95 | 89 | 117 | Moderate aggregation at initial; Stable at 24 hours |

| | | | | | |
|---|---|---|---|---|---|
| *All formulations were taken at initial time except for Lethicin and Lysozyme, wherein the initial particle size was measured at 24 hr. | | | | | |

The results demonstrate that meloxicam can be formulated into stable nanoparticulate compositions suitable for IV administration with each of the surface stabilizers shown in Table 2, as all of the formulations have a particle size suitable for injectable compositions. Nanoparticulate compositions shown in Table 2 had mean particles sizes ranging from 95 to 227 nm, with D50 and D90 sizes ranging from 89 nm to 227 nm and 117 nm to 322 nm, respectively.

### Example 4

The purpose of this example was to test *in vivo* the nanoparticulate meloxicam compositions of the invention.

Four male and four female Beagle dogs were fasted overnight. In addition, each dog was fasted for four (4) hours post dose. Each dog received three different meloxicam formualtions, which are described in more detail below. Formulation #1 was a liquid dispersion ofnanoparticulate meloxicam particles; Formulation #2 was a lyophilized table of nanoparticulate meloxicam particles; and Formulation #3 was a MOBIC® 7.5 mg Tablet (Boehringer Ingelheim Pharmaceuticals, Inc.).

### Summary of Formulations Used in the Protocol

### Formulation # 1. (liquid dispersion)

8.0 g meloxicam (Unichem Laboratories, Ltd.) was added to a solution containing 1.6 g F127 NF Lutrol® (BASF) and 70.4 g water. Lutrol® F127, also known as Poloxamer 407, is a block polymer consisting of 73% of polyethylene glycol and 27% polypropylene glycol. This mixture was then milled in a DYNO®-Mill KDL (Willy A. Bachofen AG, Maschinenfabrik, Basel, Switzerland) at 4200 RPM with PolyMill®-500 polymeric media for 160 minutes. The mean (weight average) final meloxicam particle size was 111 nm, as measured on a Horiba LB-910 particle size analyzer (Horiba Instruments, Irvine, CA).

5 grams of the meloxicam dispersion was then added to 45 grams of water to give a final concentration of 1% meloxicam.

### Formulation # 2 (lyophilzed wafers)

A "fast melt" lyophilized dosage form was prepared from a nanoparticulate dispersion of meloxicam to see how the lyophilization and reconstitution process affected the pharmacokinetic data.

8.0 g meloxicam (Unichem Laboratories, Ltd.) was added to a solution containing 2.4 g polyvinylpyrrolidone (PVP) Plasdone® K29/32 (ISP Technologies), 1.6 g docusate sodium (Cytec), and 68 g water. This mixture was then milled in a DYNO-Mill KDL (Willy A. Bachofen AG, Maschinenfabrik, Basel, Switzerland) at 4200 RPM with PolyMill®-500 polymeric media for 150 minutes.

The mean particle size of the meloxicam dispersion following milling was 101 nm, as measured on a Horiba LA-910 particle size analyzer.

1.5 grams of the dispersion was then added to 4.0 grams Mannitol USP/NF (Specturm), 1.2 grams Pullulan (Hayashibara), 0.8 grams Glycerol USP (Spectrum), and 32.5 grams of water.

A wafer tray was then filled by adding 2 grams of the diluted dispersion to each 2.5cc well and the wafer tray was then placed in a lyophilizer for 48 hours to produce the final lyophilized wafer dosage form.

The meloxicam particle size in the lyophilized wafers appears stable. After 2.5 months the reconstituted mean particle size of the meloxicam particles was 111 nm.

### Formulation # 3 (tablet)

MOBIC® Tablets (Boehringer Ingelheim), 7.5 mg.

### Dog Study Protocol

In Phase 1, each dog received a single oral gavage dose of 7.5 mg meloxicam (0.75 ml of a 10 mg / ml Formulation #1), followed by an approximately 10 mL tap water flush of the gavage tube.

In Phase 2, after a 7-day washout period, the same eight dogs received a 7.5 mg dose of meloxicam as a single lyophilized wafer (Formulation #2).

In Phase 3, after a 7-day washout period, the same 8 dogs received a single 7.5 mg tablet of MOBIC® (lot # 251586N) (Formulation #3).

### Results

Blood samples were collected and processed to plasma at the conclusion of each phase as follows: Blood samples (approximately 1 mL) were drawn at specified time points into tubes containing potassium EDTA (blood collected predose and at 0.167, 0.333, 0.5, 0.75, 1, 1.5, 2, 2.5, 3, 4, 6, 8, 12, 16, 24, and 48 hours postdose). The samples were placed on wet ice following collection. Plasma was separated and stored frozen in tubes containing potassium EDTA at approximately -20°C until shipped to the Sponsor-designated laboratory. The Cₘₐₓ, Tₘₐₓ, and AUC for the three different formulations is shown below in Table 3.

| **TABLE 4** | | | |
|---|---|---|---|
| **Formulation** | **Cmax (µg/mL)** | **Tmax (hours)** | **AUC** |
| 1 (liquid dispersion) | 3.499 | 0.750 | 118.225 |
| 2 (lyophilized wafer) | 3.420 | 1.292 | 106.642 |
| 3 (MOBIC®) | 2.768 | 3.375 | 99.870 |

Both the liquid dispersion of nanoparticulate meloxicam (Formulation #1) and the lyophilized wafer of nanoparticulate meloxicam (Formulation #2) showed a faster onset time and a larger Cₘₐₓ than the commercial MOBIC® tablet. In addition, the smaller particle sizes of the nanoparticulate meloxicam formulations resulted in faster dissolution, thereby producting a much shorter Tₘₐₓ.(0.75 and 1.3 hours, respectively, for Formulations #1 and #2, as compared to 3.4 hours for Formulation #3). *See* Figure 1.

It will be apparent to those skilled in the art that various modifications and variations can be made in the methods and compositions of the present invention without departing from the spirit or scope of the invention. Thus, it is intended that the present invention cover the modifications and variations of this invention provided they come within the scope of the appended claims and their equivalents.
The following pages 47 to 64 contain preferred embodiments. Accordingly, the term "claim" as used therein refers to such a "preferred embodiment".
1. A stable meloxicam nanoparticulate composition comprising:
   (a) particles of meloxicam or a salt thereof; and
   (b) at least one surface stabilizer,
   wherein the meloxicam particles have an effective average particle size of less than about 2000 nm.
2. The composition of claim 1, wherein the meloxicam is selected from the group consisting of a crystalline phase, an amorphous phase, a semi-crystalline phase, a semi-amorphous phase, and mixtures thereof.
3. The composition of claim 1 or claim 2, wherein the effective average particle size of the nanoparticulate meloxicam particles is selected from the group consisting of less than about 1500 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.
4. The composition of any one of claims 1 to 3, wherein the composition is formulated for administration selected from the group consisting of oral, pulmonary, rectal, opthalmic, colonic, parenteral, intracisternal, intravaginal, intraperitoneal, local, buccal, nasal, and topical administration.
5. The composition of any one of claims 1 to 4, wherein the composition is formulated into a dosage form selected from the group consisting of liquid dispersions, gels, aerosols, ointments, creams, controlled release formulations, fast melt formulations, lyophilized formulations, tablets, capsules, delayed release formulations, extended release formulations, pulsatile release formulations, and mixed immediate release and controlled release formulations.
6. The composition of any one of claims 1 to 5, wherein the composition further comprises one or more pharmaceutically acceptable excipients, carriers, or a combination thereof.
7. The composition of any one of claims 1 to 6, wherein the meloxicam is present in an amount selected from the group consisting of from about 99.5% to about 0.001%, from about 95% to about 0.1%, and from about 90% to about 0.5%, by weight, based on the total combined weight of the meloxicam and at least one surface stabilizer, not including other excipients.
8. The composition of any one of claims 1 to 7, wherein the at least one surface stabilizer is present in an amount selected from the group consisting of from about 0.01% to about 99.5% by weight, from about 0.1% to about 95% by weight, and from about 0.5% to about 90% by weight, based on the total combined dry weight of meloxicam and at least one surface stabilizer, not including other excipients.
9. The composition of any one of claims 1 to 8, comprising at least one primary surface stabilizer and at least one secondary surface stabilizer.
10. The composition of any one of claims 1 to 9, wherein the surface stabilizer is selected from the group consisting of an anionic surface stabilizer, a cationic surface stabilizer, and an ionic surface stabilizer.
11. The composition of any one of claims 1 to 10, wherein at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, C₁₈H₃₇CH₂C(O)N(CH₃)-CH₂(CHOH)₄(CH₂OH)₂, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, PEG-vitamin E, and random copolymers of vinyl acetate and vinyl pyrrolidone.
12. The composition of claim 10, wherein at least one cationic surface stabilizer is selected from the group consisting of a polymer, a biopolymer, a polysaccharide, a cellulosic, an alginate, a nonpolymeric compound, and a phospholipid.
13. The composition of claim 10, wherein the surface stabilizer is cationic and is selected from the group consisting of cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10^{™}, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts; amines, amine salts, amine oxides, imide azolinium salts, protonated quaternary acrylamides, methylated quaternary polymers, and cationic guar.
14. The composition of any one of claims 1 to 13, wherein the Tₘₐₓ of the composition, when assayed in the plasma of the mammalian subject, is selected from the group consisting of less than about 5 hours, less than about 4 hours, less than about 3 hours, less than about 2 hours, less than about 1 hour, less than less than about 50 minutes, less than about 45 minutes, less than about 40 minutes, less than about 35 minutes, less than about 30 minutes, less than about 20 minutes, less than about 15 minutes, less than about 10 minutes, and less than about 5 minutes.
15. The composition of any one of claims 1 to 14, wherein in comparative pharmacokinetic testing with a standard non-nanoparticulate commercial formulation of meloxicam, the composition of claim 1 exhibits a Tₘₐₓ selected from the group consisting of less than about 90%, less than about 80%, less than about 70%, less than about 50%, less than about 30%, less than about 25%, less than about 20%, less than about 15%, and less than about 10% of the Tₘₐₓ exhibited by the standard commercial meloxicam formulation.
16. The composition of any one of claims 1 to 15, wherein the Cₘₐₓ of the composition, when assayed in the plasma of the mammalian subject, is selected from the group consisting of greater than about 1 µg/mL, greater than about 3 µg/mL, greater than about 5 µg/mL, greater than about 10 µg/mL, and greater than about 1 5 µg/mL.
17. The composition of any one of claims 1 to 16, wherein in comparative pharmacokinetic testing with a standard non-nanoparticulate commercial formulation of meloxicam, the composition of claim 1 exhibits a Cₘₐₓ selected from the group consisting of greater than about 20%, greater than about 40%, greater than about 60%, greater than about 80%, greater than about 100%, greater than about 140%, greater than about 180%, greater than about 200%, greater than about 240%, greater than about 280%, greater than about 300%, greater than about 340%, greater than about 380%, and greater than about 400% of the Cₘₐₓ exhibited by a standard commercial meloxicam formulation.
18. The composition of any one of claims 1 to 17, additionally comprising a meloxicam composition having an effective average particle size which is greater than about 2 microns.
19. The composition of any one of claims 1 to 18, additionally comprising at least one additional nanoparticulate meloxicam composition, having an effective average particle size of less than about 2 microns, wherein said additional nanoparticulate meloxicam composition has an effective average particle size which is different than particle size of the nanoparticulate meloxicam composition of claim 1.
20. The composition of any one of claims 1 to 19, additionally comprising at least one non-meloxicam active agent selected from the group consisting of proteins, peptides, nucleotides, anti-obesity drugs, nutraceuticals, dietary supplements, carotenoids, corticosteroids, elastase inhibitors, anti-fungals, alkylxanthine, oncology therapies, anti-emetics, analgesics, opioids, antipyretics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products, blood substitutes, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio- pharmaceuticals, sex hormones, anti-allergic agents, stimulants, anoretics, sympathomimetics, thyroid agents, Mu receptor antagonists, Kappa receptor antagonists, non-narcotic analgesics, monoamine uptake inhibitors, adenosine regulating agents, cannabinoid derivatives, Substance P antagonists, neurokinin-1 receptor antagonists, and sodium channel blockers.
21. The composition of claim 20, wherein said nutraceutical is selected from the group consisting of lutein, folic acid, fatty acids, fruit extracts, vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish oils, marine animal oils, and probiotics.
22. The composition of claim 20, wherein said anti-inflammatory agent is a COX-2 inhibitor selected from the group consisting of celecoxib, rofecoxib, valdecoxib, parecoxib, MK-966, etoricoxib, 4-[5-(4-chlorophenyl)-3-(trifluoromethyl)-1H-pyrazol-1-yl)] benzenesulfonamide, N-(2-cyclohexyloxy-4-nitrophenyl)methane sulfonamide, methyl sulfone spiro(2.4)hept-5-ene I, SC-57666, celexcoxib, SC-558, SC-560, etodolac, 5,5-dimethyl-3-(3-fluorophenyl)-4-(4-methylsulfonyl)phenyl 2(5H)-furanone, MK-476, L-745337, L-761066, L-761000, L-748780, L-748731, 5-Bromo-2-(4-fluorophenyl)-3-(4-(methylsulfonyl)phenyl, 1-(7-tert.-butyl-2,3-dihydro-3,3-dimethylbenzo(b)furan-5-yl)-4-cyclopropylbutan-1-one, 3-formylamino-7-methylsulfonylamino-6-phenoxy-4H-1-benzopyran-4-one, BF 389, PD 136005, PD 142893, PD 145065, flurbiprofen, nimesulide, nabumetone, flosulide, piroxicam, dicofenac, COX-189, D 1367, 4 nitro 2 phenoxymethane sulfonanilide, (3 benzoyldifluoromethane sulfonanilide, diflumidone), JTE-522, 4'-Acetyl-2'-(2,4-difluorophenoxy)methanesulfonanilide, FK 867, FR 115068, GR 253035, RWJ 63556, RWJ 20485, ZK 38997, (E)-(5)-(3,5-di-tert-butyl-4-hydroxybenzylidene)-2-ethyl-1,2-isothiazolidine-1,1-dioxide indomethacin, CL 1004, RS 57067, RS 104894, SC 41930, SB 205312, SKB 209670, and Ono 1078.
23. The composition of claim 20, wherein said non-meloxicam active agent is selected from the group consisting of aceclofenac, acemetacin, e-acetamidocaproic acid, acetaminophen, acetaminosalol, acetanilide, acetylsalicylic acid, S-adenosylmethionine, alclofenac, alfentanil, allylprodine, alminoprofen, aloxiprin, alphaprodine, aluminum bis(acetylsalicylate), amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, bendazac, benorylate, benoxaprofen, benzpiperylon, benzydamine, benzylmorphine, bermoprofen, bezitramide, α-bisabolol, bromfenac, p-bromoacetanilide, 5-bromosalicylic acid acetate, bromosaligenin, bucetin, bucloxic acid, bucolome, bufexamac, bumadizon, buprenorphine, butacetin, butibufen, butophanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac sodium, difenamizole, difenpiramide, diflunisal, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, p-lactophenetide, lefetamine, levorphanol, lofentanil, lonazolac, lomoxicam, loxoprofen, lysine acetylsalicylate, magnesium acetylsalicylate, meclofenamic acid, mefenamic acid, meperidine, meptazinol, mesalamine, metazocine, methadone hydrochloride, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paranyline, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamide, salicin, salicylamide, salicylamide o-acetic acid, salicylsulfuric acid, salsalte, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, viminol, xenbucin, ximoprofen, zaltoprofen, and zomepirac.
24. The composition of any one of claims 20, 21, 22, or 23, wherein at least one the non-meloxicam active agents has an effective average particle size of less than about 2 microns.
25. The composition of any of 20, 21, 22, or 23, wherein at least one the non-meloxicam active agents is a conventional particle sized active agent.
26. Use of the composition according to any of claims 1-25 for preparation of a medicament.
27. The use of claim 26, wherein the medicament is useful in treating a condition selected from the group consisting of conditions in which NSAIDs are contraindicated, arthritic disorders, gastrointestinal conditions, inflammatory conditions, pulmonary inflammation, opthalmic diseases, central nervous systems disorders, pain, fever, inflammation-related cardiovascular disorders, angiogenesis-related disorders, benign tumors, malignant tumors, adenomatous polyps, endometriosis, osteoporosis, dysmenorrhea, premature labor, asthma, fibrosis which occurs with radiation treatment, eosinophil-related disorders, pyrexia, bone resorption, nephrotoxicity, hypotension, arthrosis, joint stiffness, kidney disease, liver disease, acute mastitis, diarrhea, colonic adenomas, bronchitis, allergic neuritis, cytomegalovirus infectivity, apoptosis, lumbago, psoriasis, eczema, acne, burns, dermatitis, ultraviolet radiation damage, allergic rhinitis, respiratory distress syndrome, and endotoxin shock syndrome.
28. The use of claim 26, wherein the medicament is useful in treating an indication in which anti-inflammatory agents, anti-angiogenesis agents, antitumorigenic agents, immunosuppressive agents, NSAIDs, COX-2 inhibitors, analgesic agents, anti-thrombotic agents, narcotics, or antifebrile agents are typically used.
27. A method of making a nanoparticulate composition comprising contacting meloxicam particles with at least one surface stabilizer for a time and under conditions sufficient to provide a nanoparticulate meloxicam composition having an effective average particle size of less than about 2000 nm.
28. The method of claim 27, wherein said contacting comprises grinding.
29. The method of claim 28, wherein said grinding comprises wet grinding.
30. The method of claim 27, wherein said contacting comprises homogenizing.
31. The method of claim 27, wherein said contacting comprises:
   (a) dissolving the meloxicam particles in a solvent;
   (b) adding the resulting meloxicam solution to a solution comprising at least one surface stabilizer; and
   (c) precipitating the solubilized meloxicam having at least one surface stabilizer associated with the surface thereof by the addition thereto of a non-solvent.
32. The method of any one of claims 27-31, wherein the meloxicam is selected from the group consisting of a crystalline phase, an amorphous phase, a semi-crystalline phase, a semi-amorphous phase, and mixtures thereof.
33. The method of any one of claims 27-32, wherein the effective average particle size of the nanoparticulate meloxicam particles is selected from the group consisting of less than about 1500 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.
34. The method of any one of claims 27-33, wherein the composition is formulated for an administration form selected from the group consisting of oral, pulmonary, rectal, opthalmic, colonic, parenteral, intracisternal, intravaginal, intraperitoneal, local, buccal, nasal, and topical administration.
35. The method of any one of claims 27-34, wherein the composition is formulated into a dosage form selected from the group consisting of liquid dispersions, gels, aerosols, ointments, creams, controlled release formulations, fast melt formulations, lyophilized formulations, tablets, capsules, delayed release formulations, extended release formulations, pulsatile release formulations, and mixed immediate release and controlled release formulations.
36. The method of any one of claims 27-35, wherein the composition further comprises one or more pharmaceutically acceptable excipients, carriers, or a combination thereof.
37. The method of any one of claims 27-36, wherein the meloxicam is present in an amount selected from the group consisting of from about 99% to about 0.001 %, from about 95% to about 0.5%, and from about 90% to about 0.5%, by weight, based on the total combined weight of the meloxicam and at least one surface stabilizer, not including other excipients.
38. The method of any one of claims 27-37, wherein at least one surface stabilizer is present in an amount selected from the group consisting of from about 0.01% to about 99.5% by weight, from about 0.1% to about 95% by weight, and from about 0.5% to about 90% by weight, based on the total combined dry weight of the meloxicam and at least one surface stabilizer, not including other excipients.
39. The method of any one of claims 27-38, comprising at least one primary surface stabilizer and at least one secondary surface stabilizer.
40. The method of any one of claims 27-39, wherein the surface stabilizer is selected from the group consisting of an anionic surface stabilizer, a cationic surface stabilizer, and an ionic surface stabilizer.
41. The method of claim 40, wherein at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, C₁₈H₃₇CH₂C(O)N(CH₃)-CH₂(CHOH)₄(CH₂OH)₂, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, PEG-vitamin E, and random copolymers of vinyl acetate and vinyl pyrrolidone.
42. The method of claim 40, wherein at least one cationic surface stabilizer is selected from the group consisting of a polymer, a biopolymer, a polysaccharide, a cellulosic, an alginate, a nonpolymeric compound, and a phospholipid.
43. The method of claim 40, wherein the surface stabilizer is cationic and is selected from the group consisting of cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10^{™}, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts; amines, amine salts, amine oxides, imide azolinium salts, protonated quaternary acrylamides, methylated quaternary polymers, and cationic guar.
44. The method of any one of claims 27-43, wherein after preparation of a first nanoparticulate meloxicam composition, a second meloxicam composition having an effective average particle size of greater than about 2 microns is combined with the first nanoparticulate meloxicam composition.
45. The method of any one of claims 27-44, wherein either prior to or subsequent to preparation of the nanoparticulate meloxicam composition, at least one non-meloxicam active agent is added to the meloxicam composition, wherein said non-meloxicam active agent is selected from the group consisting of proteins, peptides, nucleotides, anti-obesity drugs, nutraceuticals, dietary supplements, carotenoids, corticosteroids, elastase inhibitors, anti-fungals, alkylxanthine, oncology therapies, anti-emetics, analgesics, opioids, antipyretics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products, blood substitutes, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio- pharmaceuticals, sex hormones, anti-allergic agents, stimulants, anoretics, sympathomimetics, thyroid agents, Mu receptor antagonists, Kappa receptor antagonists, non-narcotic analgesics, monoamine uptake inhibitors, adenosine regulating agents, cannabinoid derivatives, Substance P antagonists, neurokinin-1 receptor antagonists, and sodium channel blockers.
46. The method of claim 45, wherein said nutraceutical is selected from the group consisting of lutein, folic acid, fatty acids, fruit extracts, vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish oils, marine animal oils, and probiotics.
47. The method of claim 45, wherein said anti-inflammatory agent is a COX-2 inhibitor selected from the group consisting of celecoxib, rofecoxib, valdecoxib, parecoxib, MK-966, etoricoxib, 4-[5-(4-chlorophenyl)-3-(trifluoromethyl)-1H-pyrazol-1-yl)]benzenesulfonamide, N-(2-cyclohexyloxy-4-nitrophenyl)methane sulfonamide, methyl sulfone spiro(2.4)hept-5-ene I, SC-57666, celexcoxib, SC-558, SC-560, etodolac, 5,5-dimethyl-3-(3-fluorophenyl)-4-(4-methylsulfonyl)phenyl 2(5H)-furanone, MK-476, L-745337, L-761066, L-761000, L-748780, L-748731, 5-Bromo-2-(4-fluorophenyl)-3-(4-(methylsulfonyl)phenyl, 1-(7-tert.-butyl-2,3-dihydro-3,3-dimethylbenzo(b)furan-5-yl)-4-cyclopropylbutan-1-one, 3-formylamino-7-methylsulfonylamino-6-phenoxy-4H-1-benzopyran-4-one, BF 389, PD 136005, PD 142893, PD 145065, flurbiprofen, nimesulide, nabumetone, flosulide, piroxicam, dicofenac, COX-189, D 1367, 4 nitro 2 phenoxymethane sulfonanilide, (3 benzoyldifluoromethane sulfonanilide, diflumidone), JTE-522, 4'-Acetyl-2'-(2,4-difluorophenoxy)methanesulfonanilide, FK 867, FR 115068, GR 253035, RWJ 63556, RWJ 20485, ZK 38997, (E)-(5)-(3,5-di-tert-butyl-4-hydroxybenzylidene)-2-ethyl-1,2-isothiazolidine-1,1-dioxide indomethacin, CL 1004, RS 57067, RS 104894, SC 41930, SB 205312, SKB 209670, and Ono 1078.
48. The method of claim 45, wherein said non-meloxicam active agent is selected from the group consisting of aceclofenac, acemetacin, e-acetamidocaproic acid, acetaminophen, acetaminosalol, acetanilide, acetylsalicylic acid, S-adenosylmethionine, alclofenac, alfentanil, allylprodine, alminoprofen, aloxiprin, alphaprodine, aluminum bis(acetylsalicylate), amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, bendazac, benorylate, benoxaprofen, benzpiperylon, benzydamine, benzylmorphine, bermoprofen, bezitramide, α-bisabolol, bromfenac, p-bromoacetanilide, 5-bromosalicylic acid acetate, bromosaligenin, bucetin, bucloxic acid, bucolome, bufexamac, bumadizon, buprenorphine, butacetin, butibufen, butophanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac sodium, difenamizole, difenpiramide, diflunisal, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, p-lactophenetide, lefetamine, levorphanol, lofentanil, lonazolac, lomoxicam, loxoprofen, lysine acetylsalicylate, magnesium acetylsalicylate, meclofenamic acid, mefenamic acid, meperidine, meptazinol, mesalamine, metazocine, methadone hydrochloride, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paranyline, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamide, salicin, salicylamide, salicylamide o-acetic acid, salicylsulfuric acid, salsalte, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, viminol, xenbucin, ximoprofen, zaltoprofen, and zomepirac.
49. The method of any of claims 45, 46, 47, or 48, wherein at least one non-meloxicam active agent has an effective average particle size of less than about 2 microns.
50. The method of any of claims 45, 46, 47, or 48, wherein at least one non-meloxicam active agent has an effective average particle size of greater than about 2 microns.

## Claims

1. A stable meloxicam composition comprising:
(a) nanoparticulate particles of meloxicam or a salt thereof, wherein the meloxicam particles have an effective average particle size of less than about 2000 nm;
(b) particles of meloxicam having an effective average particle size which is less or greater than about 2 microns; wherein said effective average particle size is different than the particle size of the nanoparticulate particles of meloxicam (a); and
(c) at least one surface stabilizer.

2. The composition of claim 1, wherein the particles of meloxicam of (b) have a pharmacokinetic profile different from that of the nanoparticulate particles of meloxicam of (a), particularly a longer Tₘₐₓ and optionally a lower Cₘₐₓ.

3. The composition of claim 1 or claim 2, wherein the meloxicam is selected from the group consisting of a crystalline phase, an amorphous phase, a semi-crystalline phase, a semi-amorphous phase, and mixtures thereof.

4. The composition of any one of claims 1 to 3, wherein the effective average particle size of the nanoparticulate particles of meloxicam is selected from the group consisting of less than about 1500 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.

5. The composition of any one of claims 1 to 4, wherein the composition is formulated:
(a) for administration selected from the group consisting of oral, pulmonary, rectal, opthalmic, colonic, parenteral, intracisternal, intravaginal, intraperitoneal, local, buccal, nasal, and topical administration; and/or
(b) into a dosage form selected from the group consisting of liquid dispersions, gels, aerosols, ointments, creams, controlled release formulations, fast melt formulations, lyophilized formulations, tablets, capsules, delayed release formulations, extended release formulations, pulsatile release formulations, and mixed immediate release and controlled release formulations.

6. The composition of any one of claims 1 to 5, wherein:
(a) the meloxicam is present in an amount selected from the group consisting of from about 99.5% to about 0.001 %, from about 95% to about 0.1 %, and from about 90% to about 0.5%, by weight, based on the total combined weight of the meloxicam and at least one surface stabilizer, not including other excipients; and/or
(b) the at least one surface stabilizer is present in an amount selected from the group consisting of from about 0.01% to about 99.5% by weight, from about 0.1 % to about 95% by weight, and from about 0.5% to about 90% by weight, based on the total combined dry weight of meloxicam and at least one surface stabilizer, not including other excipients.

7. The composition of any one of claims 1 to 6, comprising at least one primary surface stabilizer and at least one secondary surface stabilizer.

8. The composition of any one of claims 1 to 7, wherein:
(a) the surface stabilizer is selected from the group consisting of an anionic surface stabilizer, a cationic surface stabilizer, and an ionic surface stabilizer; and/or
(b) at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, C₁₈H₃₇CH₂C(O)N(CH₃)-CH₂(CHOH)₄(CH₂OH)₂, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, PEG-vitamin E, random copolymers of vinyl acetate and vinyl pyrrolidone, cationic polymers, cationic biopolymers, cationic polysaccharides, cationic cellulosics, cationic alginate, cationic nonpolymeric compounds, cationic phospholipids, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅ dimethyl hydroxyethyl ammonium chloride, C₁₂₋₁₅ dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10^{™}, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts; amines, amine salts, amine oxides, imide azolinium salts, protonated quaternary acrylamides, methylated quaternary polymers, and cationic guar.

9. The composition of any one of claims 1 to 8, wherein:
(a) the Tₘₐₓ of the nanoparticulate particles of meloxicam of (a), when assayed in the plasma of the mammalian subject, is selected from the group consisting of less than about 5 hours, less than about 4 hours, less than about 3 hours, less than about 2 hours, less than about 1 hour, less than less than about 50 minutes, less than about 45 minutes, less than about 40 minutes, less than about 35 minutes, less than about 30 minutes, less than about 20 minutes, less than about 15 minutes, less than about 10 minutes, and less than about 5 minutes; and/or
(b) the Cₘₐₓ of the nanoparticulate particles of meloxicam of (a), when assayed in the plasma of the mammalian subject, is selected from the group consisting of greater than about 1 µg/mL, greater than about 3 µg/mL, greater than about 5 µg/mL, greater than about 10 µg/mL, and greater than about 15 µg/mL.

10. The composition of any one of claims 1 to 9, wherein in comparative pharmacokinetic testing with a standard non-nanoparticulate commercial formulation of meloxicam, the nanoparticulate particles of meloxicam of (a) exhibits:
(a) a Tₘₐₓ selected from the group consisting of less than about 90%, less than about 80%, less than about 70%, less than about 50%, less than about 30%, less than about 25%, less than about 20%, less than about 15%, and less than about 10% of the Tₘₐₓ exhibited by the standard commercial meloxicam formulation; and/or
(b) a Cₘₐₓ selected from the group consisting of greater than about 20%, greater than about 40%, greater than about 60%, greater than about 80%, greater than about 100%, greater than about 140%, greater than about 180%, greater than about 200%, greater than about 240%, greater than about 280%, greater than about 300%, greater than about 340%, greater than about 380%, and greater than about 400% of the Cₘₐₓ exhibited by a standard commercial meloxicam formulation.

11. The composition of any one of claims 1 to 10 additionally comprising at least one non-meloxicam active agent selected from the group consisting of proteins, peptides, nucleotides, anti-obesity drugs, nutraceuticals, dietary supplements, carotenoids, corticosteroids, elastase inhibitors, anti-fungals, alkylxanthine, oncology therapies, anti-emetics, analgesics, opioids, antipyretics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products, blood substitutes, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio- pharmaceuticals, sex hormones, anti-allergic agents, stimulants, anoretics, sympathomimetics, thyroid agents, Mu receptor antagonists, Kappa receptor antagonists, non-narcotic analgesics, monoamine uptake inhibitors, adenosine regulating agents, cannabinoid derivatives, Substance P antagonists, neurokinin-1 receptor antagonists, and sodium channel blockers, particularly wherein:
(i) the nutraceutical is selected from the group consisting of lutein, folic acid, fatty acids, fruit extracts, vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish oils, marine animal oils, and probiotics; and/or
(ii) the anti-inflammatory agent is a COX-2 inhibitor selected from the group consisting of celecoxib, rofecoxib, valdecoxib, parecoxib, MK-966, etoricoxib, 4-[5-(4-chlorophenyl)-3-(trifluoromethyl)-1 H-pyrazol-1-yl)] benzenesulfonamide, N-(2-cyclohexyloxy-4-nitrophenyl)methane sulfonamide, methyl sulfone spiro(2.4)hept-5-ene I, SC-57666, celexcoxib, SC-558, SC-560, etodolac, 5,5-dimethyl-3-(3-fluorophenyl)-4-(4-methylsulfonyl)phenyl 2(5H)-furanone, MK-476, L-745337, L-761066, L-761000, L-748780, L-748731, 5-Bromo-2-(4-fluorophenyl)-3-(4-(methylsulfonyl)phenyl, 1-(7-tert.-butyl-2,3-dihydro-3,3-dimethylbenzo(b)furan-5-yl)-4-cyclopropylbutan-1-one, 3-formylamino-7-methylsulfonylamino-6-phenoxy-4H-1- benzopyran-4-one, BF 389, PD 136005, PD 142893, PD 145065, flurbiprofen, nimesulide, nabumetone, flosulide, piroxicam, dicofenac, COX-189, D 1367, 4 nitro 2 phenoxymethane sulfonanilide, (3 benzoyldifluoromethane sulfonanilide, diflumidone), JTE-522, 4'-Acetyl-2'-(2,4-difluorophenoxy)methanesulfonanilide, FK 867, FR 115068, GR 253035, RWJ 63556, RWJ 20485, ZK 38997, (E)-(5)-(3,5-di-tert-butyl-4-hydroxybenzylidene)-2-ethyl-1,2-isothiazolidine-1,1-dioxide indomethacin, CL 1004, RS 57067, RS 104894, SC 41930, SB 205312, SKB 209670, and Ono 1078; and/or
(iii) the non-meloxicam active agent is selected from the group consisting of aceclofenac, acemetacin, e-acetamidocaproic acid, acetaminophen, acetaminosalol, acetanilide, acetylsalicylic acid, S-adenosylmethionine, alclofenac, alfentanil, allylprodine, alminoprofen, aloxiprin, alphaprodine, aluminum bis(acetylsalicylate), amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, bendazac, benorylate, benoxaprofen, benzpiperylon, benzydamine, benzylmorphine, bermoprofen, bezitramide, α-bisabolol, bromfenac, p-bromoacetanilide, 5-bromosalicylic acid acetate, bromosaligenin, bucetin, bucloxic acid, bucolome, bufexamac, bumadizon, buprenorphine, butacetin, butibufen, butophanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac sodium, difenamizole, difenpiramide, diflunisal, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, p-lactophenetide, lefetamine, levorphanol, lofentanil, lonazolac, lomoxicam, loxoprofen, lysine acetylsalicylate, magnesium acetylsalicylate, meclofenamic acid, mefenamic acid, meperidine, meptazinol, mesalamine, metazocine, methadone hydrochloride, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paranyline, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamide, salicin, salicylamide, salicylamide o-acetic acid, salicylsulfuric acid, salsalte, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, viminol, xenbucin, ximoprofen, zaltoprofen, and zomepirac.

12. The composition of claim 11, wherein at least one non-meloxicam active agent:
(a) has an effective average particle size of less than about 2 microns; and/or
(b) is a conventional particle sized active agent.

13. The composition of any one of claims 1 to 12, wherein the composition further comprises one or more pharmaceutically acceptable excipients, carriers, or a combination thereof.

14. The composition of any one of claims 1 to 13 for use as a medicament, particularly wherein the medicament is useful in treating:
(a) a condition selected from the group consisting of conditions in which NSAIDs are contraindicated, arthritic disorders, gastrointestinal conditions, inflammatory conditions, pulmonary inflammation, opthalmic diseases, central nervous systems disorders, pain, fever, inflammation-related cardiovascular disorders, angiogenesis-related disorders, benign tumors, malignant tumors, adenomatous polyps, endometriosis, osteoporosis, dysmenorrhea, premature labor, asthma, fibrosis which occurs with radiation treatment, eosinophil-related disorders, pyrexia, bone resorption, nephrotoxicity, hypotension, arthrosis, joint stiffness, kidney disease, liver disease, acute mastitis, diarrhea, colonic adenomas, bronchitis, allergic neuritis, cytomegalovirus infectivity, apoptosis, lumbago, psoriasis, eczema, acne, burns, dermatitis, ultraviolet radiation damage, allergic rhinitis, respiratory distress syndrome, and endotoxin shock syndrome; and/or
(b) an indication in which anti-inflammatory agents, anti-angiogenesis agents, antitumorigenic agents, immunosuppressive agents, NSAIDs, COX-2 inhibitors, analgesic agents, anti-thrombotic agents, narcotics, or antifebrile agents are typically used.

15. Use of the composition according to any of claims 1 to 13 for preparation of a medicament, particularly wherein the medicament is useful in treating:
(a) a condition selected from the group consisting of conditions in which NSAIDs are contraindicated, arthritic disorders, gastrointestinal conditions, inflammatory conditions, pulmonary inflammation, opthalmic diseases, central nervous systems disorders, pain, fever, inflammation-related cardiovascular disorders, angiogenesis-related disorders, benign tumors, malignant tumors, adenomatous polyps, endometriosis, osteoporosis, dysmenorrhea, premature labor, asthma, fibrosis which occurs with radiation treatment, eosinophil-related disorders, pyrexia, bone resorption, nephrotoxicity, hypotension, arthrosis, joint stiffness, kidney disease, liver disease, acute mastitis, diarrhea, colonic adenomas, bronchitis, allergic neuritis, cytomegalovirus infectivity, apoptosis, lumbago, psoriasis, eczema, acne, burns, dermatitis, ultraviolet radiation damage, allergic rhinitis, respiratory distress syndrome, and endotoxin shock syndrome; and/or
(b) an indication in which anti-inflammatory agents, anti-angiogenesis agents, antitumorigenic agents, immunosuppressive agents, NSAIDs, COX-2 inhibitors, analgesic agents, anti-thrombotic agents, narcotics, or antifebrile agents are typically used.
